(19) **Europäisches Patentamt / European Patent Office / Office européen des brevets**

(11) **EP 4 006 110 A1**

(12) **EUROPEAN PATENT APPLICATION**
published in accordance with Art. 153(4) EPC

(43) Date of publication:
**01.06.2022 Bulletin 2022/22**

(21) Application number: **20848223.2**

(22) Date of filing: **31.07.2020**

(51) International Patent Classification (IPC):
$C09C\ 1/00^{(2006.01)}$   $C09C\ 1/02^{(2006.01)}$
$C09C\ 1/04^{(2006.01)}$   $C09C\ 1/24^{(2006.01)}$

(52) Cooperative Patent Classification (CPC):
**A61K 8/02; A61K 8/19; A61K 8/26; A61K 8/27;**
**A61K 8/28; A61K 8/29; A61K 8/73; A61Q 1/00;**
**A61Q 5/00; A61Q 17/04; A61Q 19/00; A61Q 19/10;**
**C09C 1/00; C09C 1/02; C09C 1/04;**   (Cont.)

(86) International application number:
**PCT/JP2020/029440**

(87) International publication number:
**WO 2021/020560 (04.02.2021 Gazette 2021/05)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **31.07.2019   JP 2019141622**
**27.04.2020   JP 2020078374**

(71) Applicant: **Nissan Chemical Corporation**
**Tokyo 103-6119 (JP)**

(72) Inventors:
• **IMOTO, Takayuki**
  **Funabashi-shi, Chiba 274-0052 (JP)**
• **KATSUYA, Mutsuhiro**
  **Funabashi-shi, Chiba 274-0052 (JP)**

(74) Representative: **dompatent von Kreisler Selting**
**Werner -**
**Partnerschaft von Patent- und Rechtsanwälten**
**mbB**
**Deichmannhaus am Dom**
**Bahnhofsvorplatz 1**
**50667 Köln (DE)**

(54) **PIGMENT INCLUDING CELLULOSE**

(57)    An object is to reduce the occurrence of aggregation of an inorganic pigment that roughens the texture and dulls the color of a cosmetic containing the inorganic pigment. Porous pigment particles are provided which include cellulose or a cellulose derivative, and an inorganic pigment as main components. Also provided are a method for producing such particles, and a cosmetic containing such porous pigment particles. The particles are resistant to aggregation and are excellently dispersed in a base material to impart a color while improving the dullness problem. The particles of the present invention are porous particles that contain cellulose and have a wrinkle-like or fold-like uneven structure on the surface thereof (that is, have an appropriate amount of pores or voids). Thus, the particles of the present invention have soft and comfortable texture and are suitably added to cosmetics that are directly applied to the skin.

Fig. 1 (a)

**(Cont. next page)**

(52) Cooperative Patent Classification (CPC): (Cont.)
**C09C 1/24; C09C 1/26; C09C 1/32; C09C 1/34;
C09C 1/36; C09C 1/40; C09C 3/10**

**Description**

Technical Field

The present invention relates to porous pigment particles containing cellulose or a cellulose derivative, to a method for producing the same, and to a cosmetic containing the particles.

Background Art

Inorganic pigments are inorganic substances used for coloring that are almost insoluble in water or organic solvents. Inorganic pigments compare unfavorably to organic pigments in aspects such as the vividness of colors, but are excellent in light resistance, heat resistance and hiding properties and are used in, for example, cosmetics for purposes such as to impart coloration properties and masking properties. While inorganic pigments have excellent solvent resistance, they are sometimes aggregated in base materials. The aggregation of inorganic pigments roughens the texture and dulls the colors of cosmetics to which the pigments are added.

Summary of Invention

Technical Problem

An object of the present invention is to reduce the occurrence of aggregation of an inorganic pigment that roughens the texture and dulls the color of a cosmetic containing the inorganic pigment.

Solution to Problem

The present inventors have diligently studied particles containing cellulose or a cellulose derivative in anticipation of the development of defocus effect (also called soft focus effect). The present inventors have then found that porous pigment particles including an inorganic pigment and cellulose or a cellulose derivative exhibit excellent dispersibility in a base material, impart a color while improving the dullness problem, are resistant to aggregation, and offer soft and comfortable texture stemming from the cellulose on the particle surface. The present invention has been completed based on the finding. Aspects of the present invention are as described below.

(1) A porous pigment particle comprising cellulose or a cellulose derivative, and an inorganic pigment as main components.
(2) The particle described in (1), which has a void ratio in the range of 5 to 50%.
(3) The particle described in (1) or (2), which has a particle size in the range of 0.1 to 500 $\mu$m.
(4) The particle described in any of (1) to (3), which has a light scattering rate in the range of 50 to 230%.
(5) The particle described in any of (1) to (4), wherein, in a measurement of a distribution of reflected light, a ratio of an intensity of the reflected light in a vicinity of a specular reflection to incident light with respect to an intensity of the reflected light in a vicinity of the incident light is in the range of 0.5:1 to 2:1.
(6) The particle described in any of (1) to (5), wherein the cellulose is crystalline cellulose.
(7) The particle described in any of (1) to (6), wherein the inorganic pigment is at least one selected from the group consisting of white pigments, color pigments, pearlescent pigments and functional pigments.
(8) The particle described in any of (1) to (7), wherein the inorganic pigment is selected from the group consisting of red iron oxide, yellow iron oxide, black iron oxide, chromium oxide, ultramarine blue, titanium oxide, zinc oxide, aluminum oxide, boron nitride and barium sulfate.
(9) The particle described in any of (1) to (8), wherein the ratio (by weight) of the cellulose or the cellulose derivative to the inorganic pigment is in the range of 1:1 to 1:20.
(10) A method for producing a porous pigment particle containing cellulose or a cellulose derivative, and an inorganic pigment as main components, the method comprising:
a step of obtaining a dispersion containing cellulose or a cellulose derivative, and an inorganic pigment, and a step of spray-drying the dispersion obtained.
(11) The production method described in (10), wherein the dispersion is obtained through physical pulverization of the cellulose or the cellulose derivative, and the inorganic pigment.
(12) The production method described in (10) or (11), wherein the concentration of solids containing the cellulose or the cellulose derivative, and the inorganic pigment in the dispersion is 0.5 to 50 mass%.
(13) The production method described in any of (10) to (12), wherein the ratio (by weight) of the cellulose or the cellulose derivative to the inorganic pigment is in the range of 1:1 to 1:20.

(14) A cosmetic comprising the particle described in any of (1) to (9), or a particle obtained by the production method described in any of (10) to (13).

Advantageous Effects of Invention

The porous pigment particles of the present invention are resistant to aggregation and are excellently dispersed in a base material to impart a color while improving the dullness problem. Specifically, the porous pigment particles provided by the present invention may offer enhanced lightness when the inorganic pigment is a white pigment, and may impart a deep color when the inorganic pigment is a colored pigment. The particles of the present invention are porous particles that contain cellulose and have a wrinkle-like or fold-like uneven structure on the surface thereof (that is, have an appropriate amount of pores or voids). Thus, the particles of the present invention have soft and comfortable texture and are suitably added to cosmetics that are directly applied to the skin. Further, the particles of the present invention have excellent optical characteristics (light scattering properties) in which incident light is uniformly scattered. Thus, the addition of the particles to cosmetics such as foundations is expected to produce the defocus effect (also called the soft focus effect).

Brief Description of Drawings

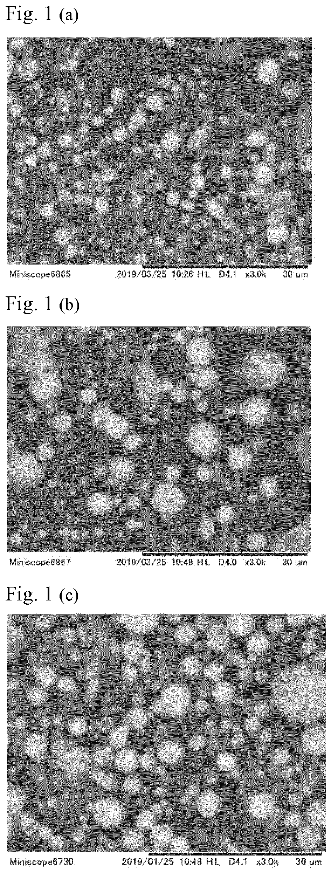

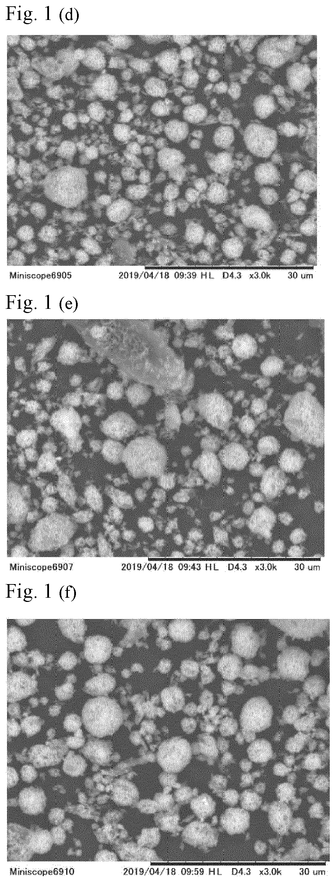

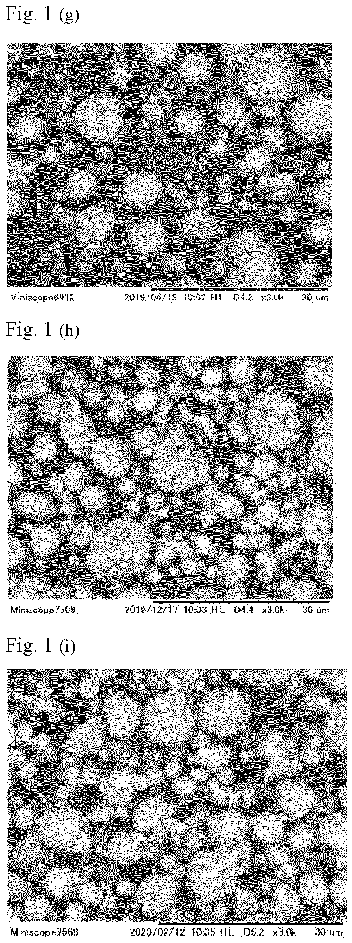

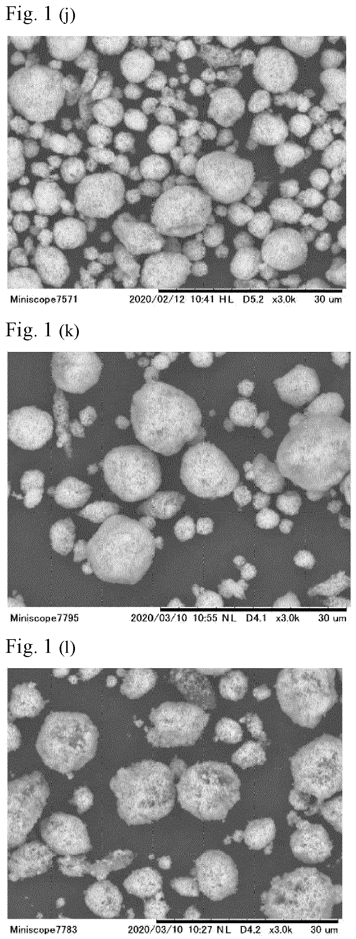

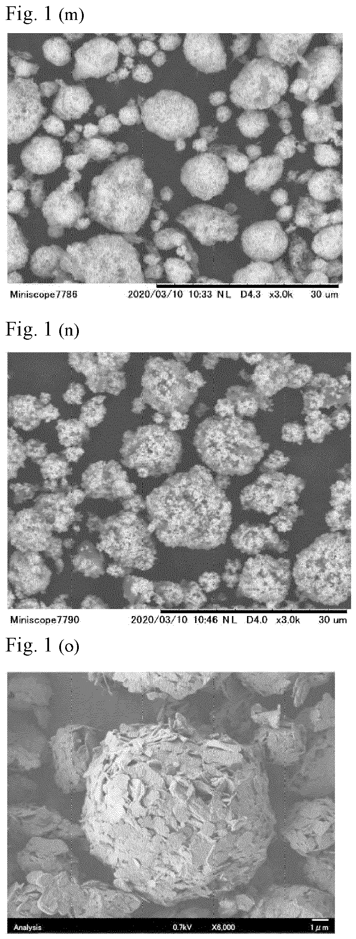

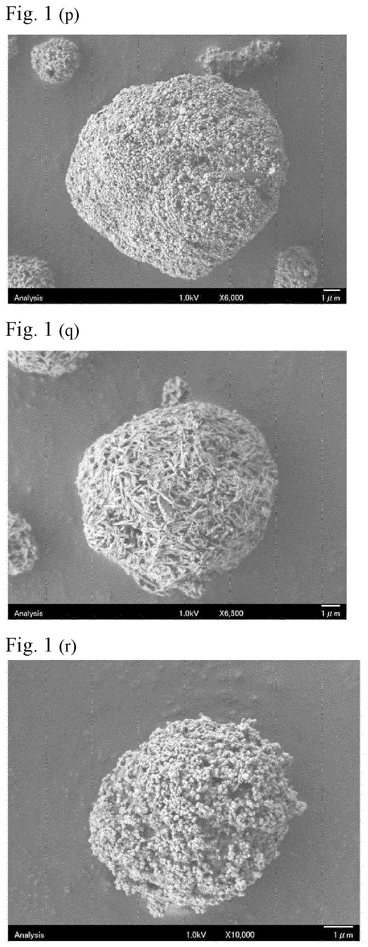

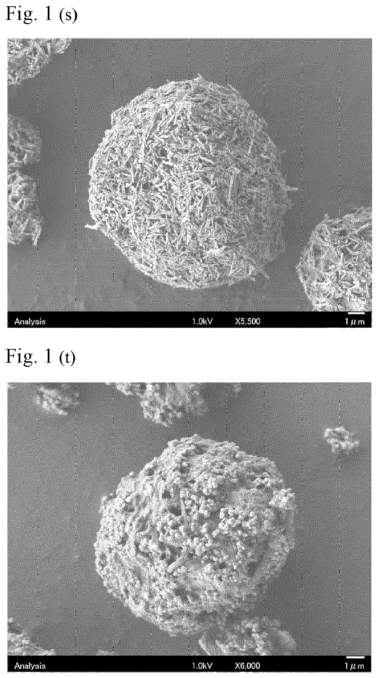

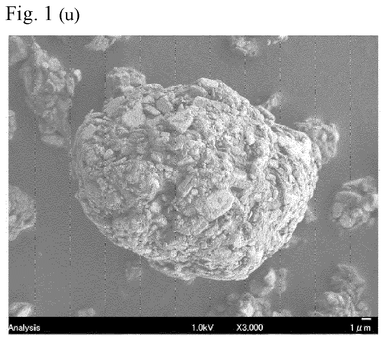

[Fig. 1] Figures (a) to (n) are scanning electron microscope (SEM) images of particles obtained in Examples 1 to 7, and 9 to 15, respectively, and Figures (o) to (u) are field emission scanning electron microscope (FE-SEM) images of particles obtained in Examples 8, 9, and 12 to 16, respectively.

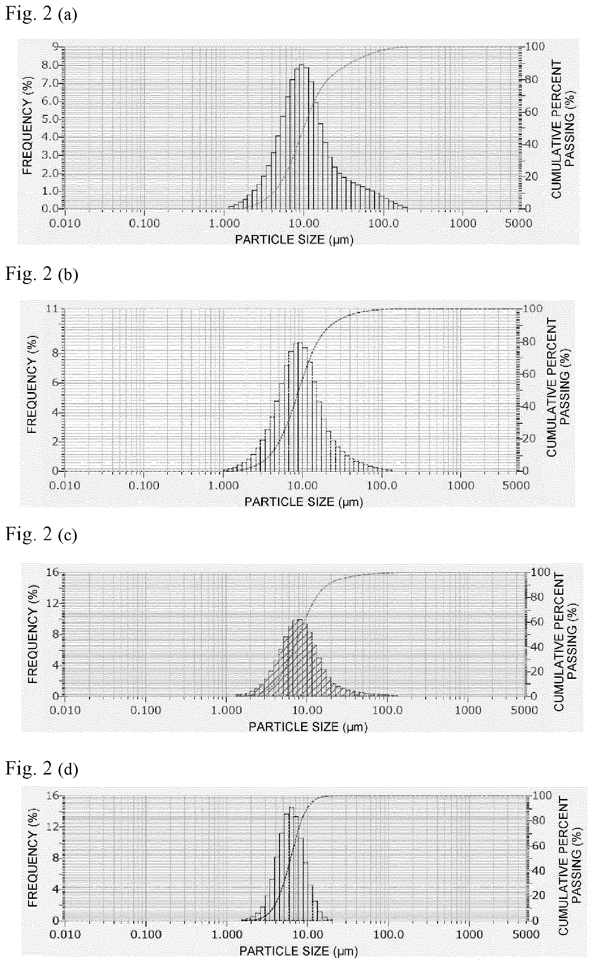

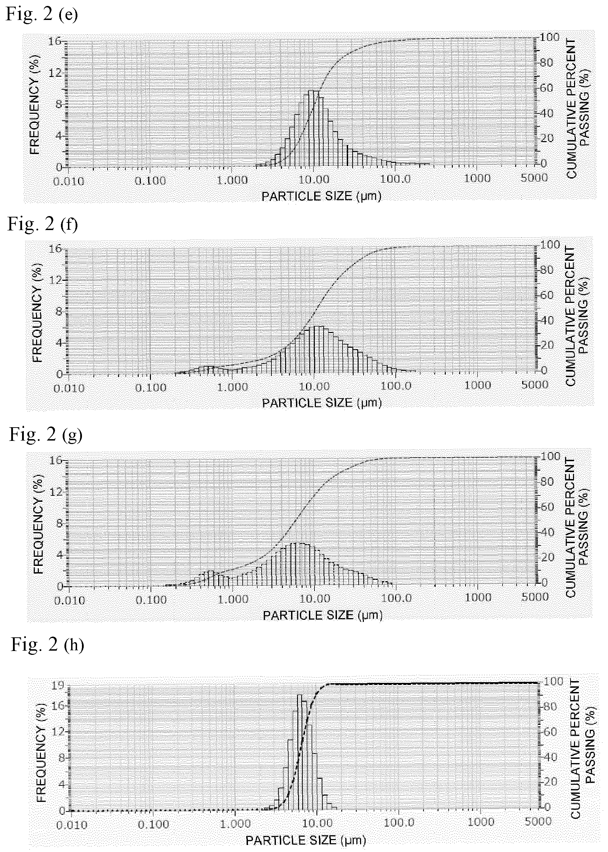

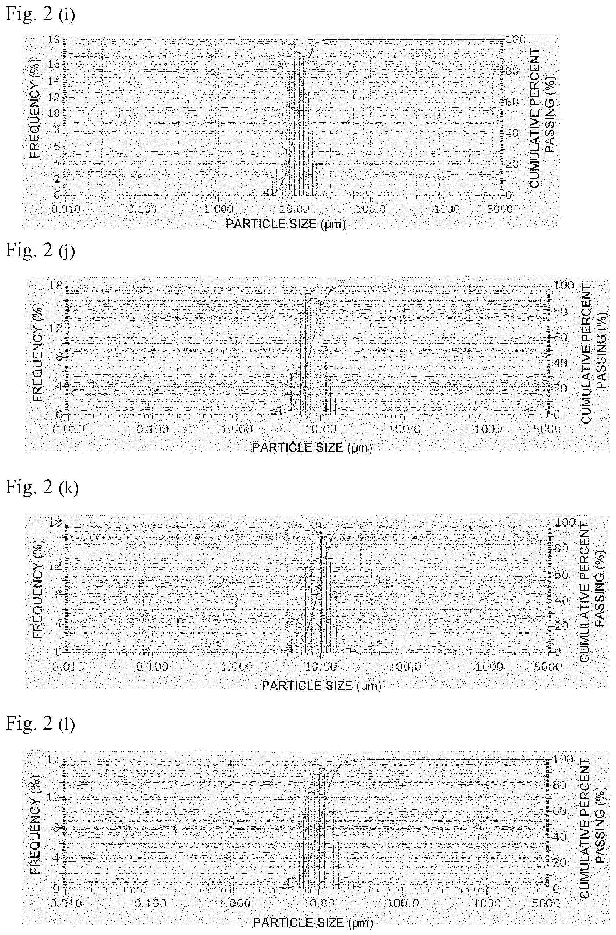

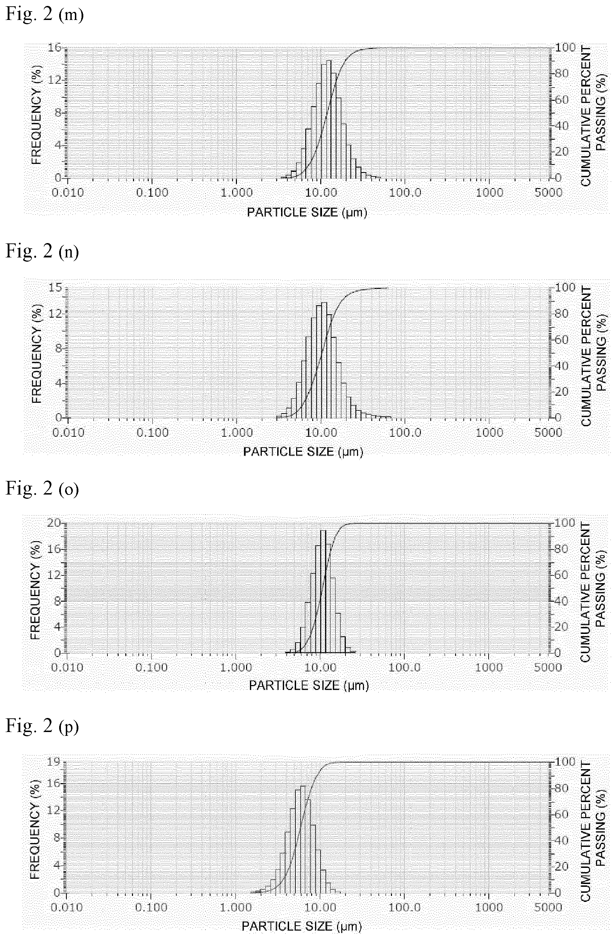

[Fig. 2] Figures (a) to (p) are volume-based particle size distribution graphs of particles obtained in Examples 1 to 16, respectively.

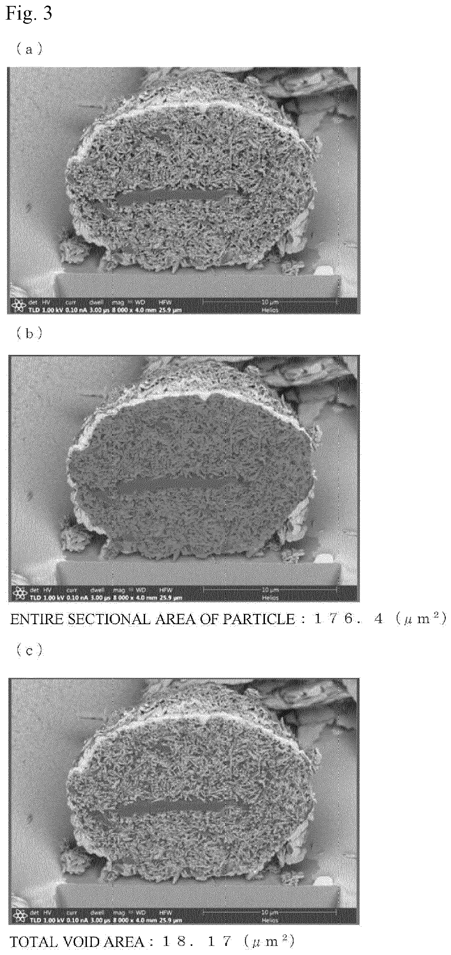

[Fig. 3] Figure (a) is a SEM image of a cross section of a particle obtained in Example 3; Figure (b) is a SEM image highlighting the entire cross section (the sectional area) of the particle used in the calculation of void ratio; and Figure (c) is a SEM image highlighting the voids in the cross section of the particle used in the calculation of void ratio.

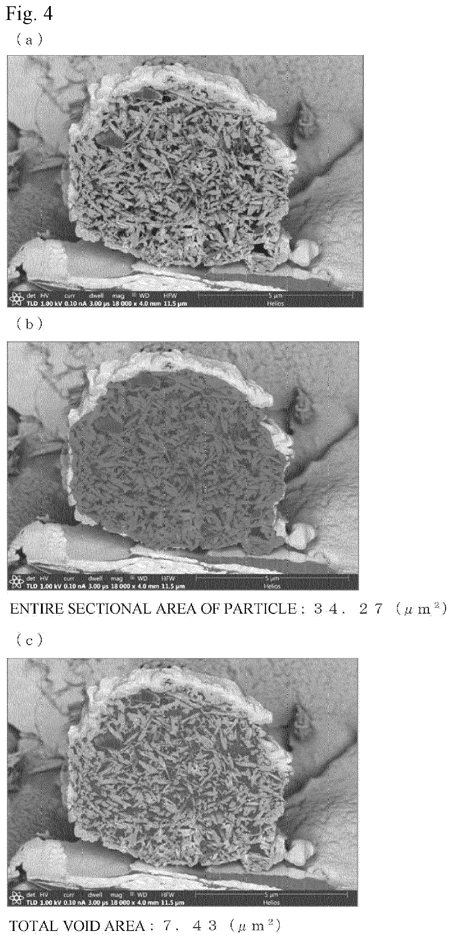

[Fig. 4] Figure (a) is a SEM image of a cross section of a particle obtained in Example 4; Figure (b) is a SEM image highlighting the entire cross section (the sectional area) of the particle used in the calculation of void ratio; and Figure (c) is a SEM image highlighting the voids in the cross section of the particle used in the calculation of void ratio.

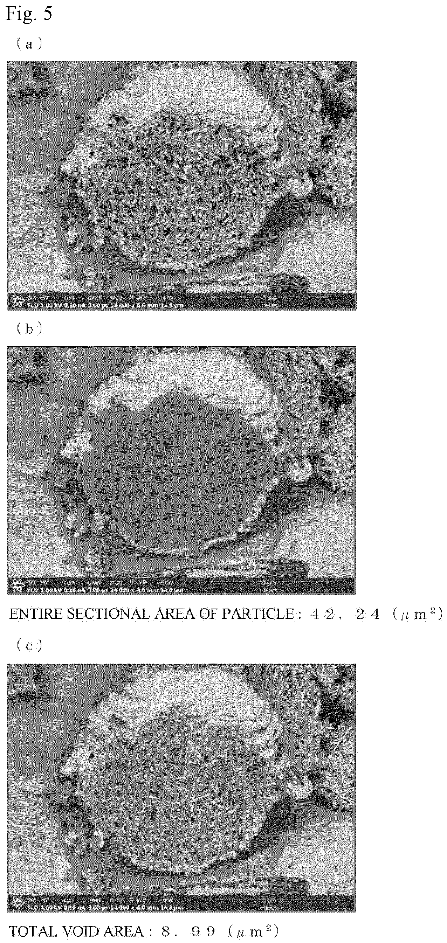

[Fig. 5] Figure (a) is a SEM image of a cross section of a particle obtained in Example 5; Figure (b) is a SEM image highlighting the entire cross section (the sectional area) of the particle used in the calculation of void ratio; and Figure (c) is a SEM image highlighting the voids in the cross section of the particle used in the calculation of void ratio.

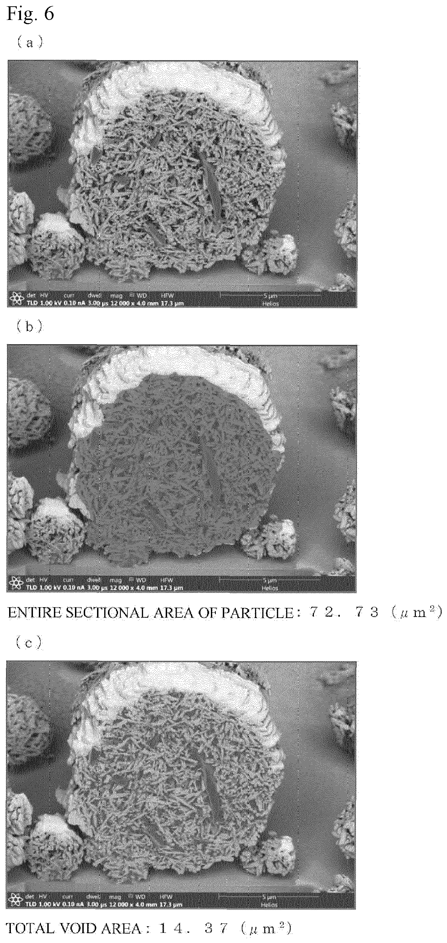

[Fig. 6] Figure (a) is a SEM image of a cross section of a particle obtained in Example 6; Figure (b) is a SEM image highlighting the entire cross section (the sectional area) of the particle used in the calculation of void ratio; and Figure (c) is a SEM image highlighting the voids in the cross section of the particle used in the calculation of void ratio.

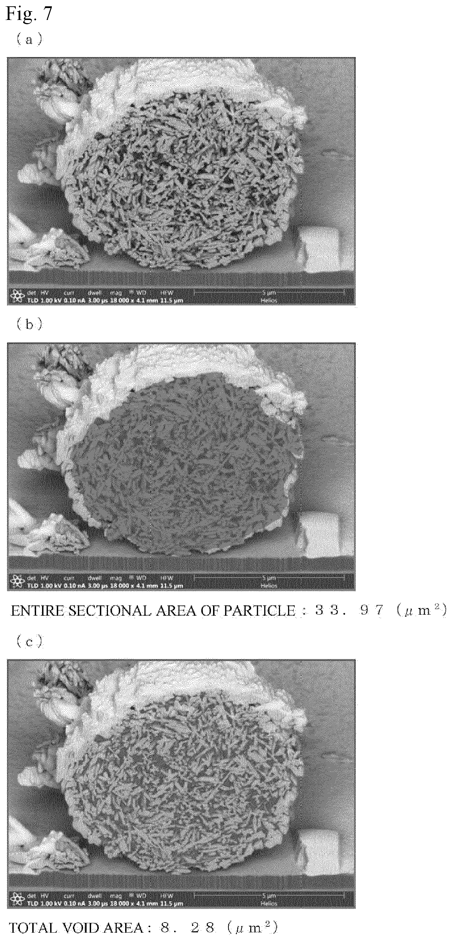

[Fig. 7] Figure (a) is a SEM image of a cross section of a particle obtained in Example 7; Figure (b) is a SEM image highlighting the entire cross section (the sectional area) of the particle used in the calculation of void ratio; and Figure (c) is a SEM image highlighting the voids in the cross section of the particle used in the calculation of void ratio.

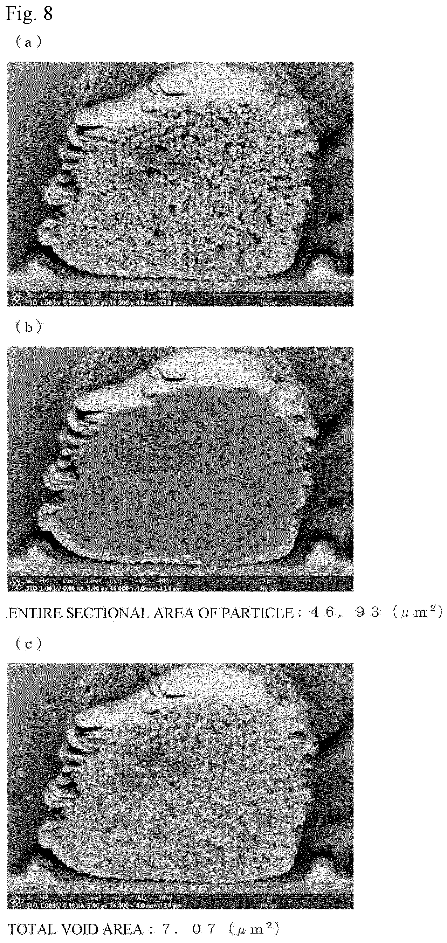

[Fig. 8] Figure (a) is a SEM image of a cross section of a particle obtained in Example 9; Figure (b) is a SEM image highlighting the entire cross section (the sectional area) of the particle used in the calculation of void ratio; and Figure (c) is a SEM image highlighting the voids in the cross section of the particle used in the calculation of void ratio.

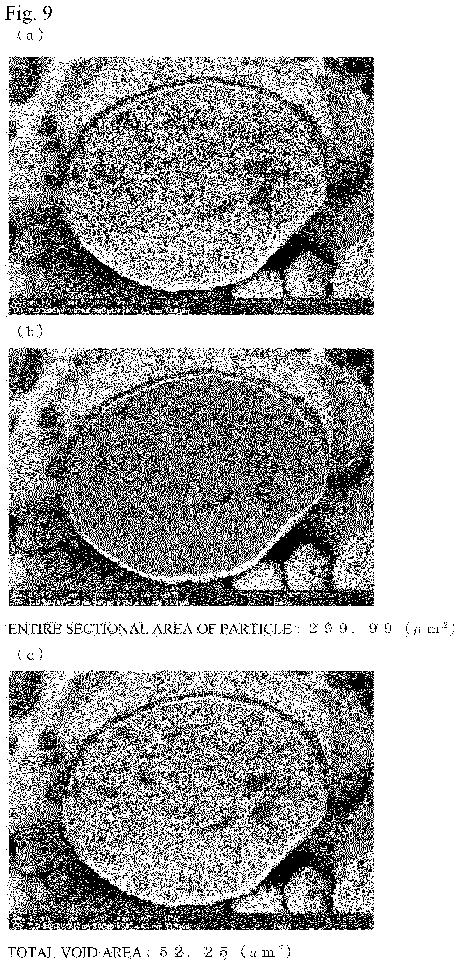

[Fig. 9] Figure (a) is a SEM image of a cross section of a particle obtained in Example 12; Figure (b) is a SEM image highlighting the entire cross section (the sectional area) of the particle used in the calculation of void ratio; and Figure (c) is a SEM image highlighting the voids in the cross section of the particle used in the calculation of void ratio.

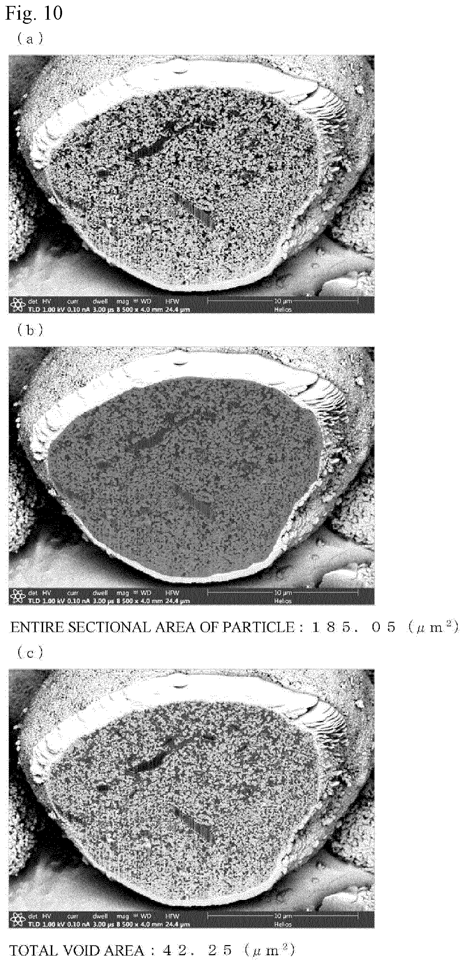

[Fig. 10] Figure (a) is a SEM image of a cross section of a particle obtained in Example 13; Figure (b) is a SEM image highlighting the entire cross section (the sectional area) of the particle used in the calculation of void ratio; and Figure (c) is a SEM image highlighting the voids in the cross section of the particle used in the calculation of void ratio.

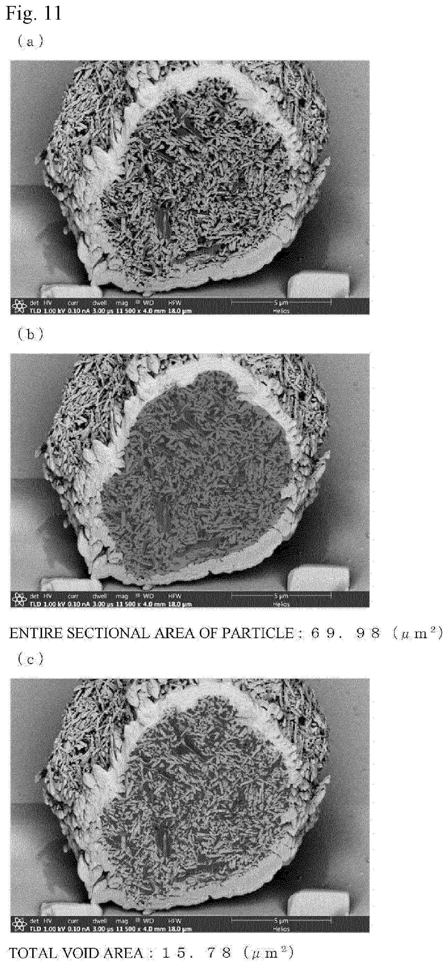

[Fig. 11] Figure (a) is a SEM image of a cross section of a particle obtained in Example 14; Figure (b) is a SEM image highlighting the entire cross section (the sectional area) of the particle used in the calculation of void ratio; and Figure (c) is a SEM image highlighting the voids in the cross section of the particle used in the calculation of void ratio.

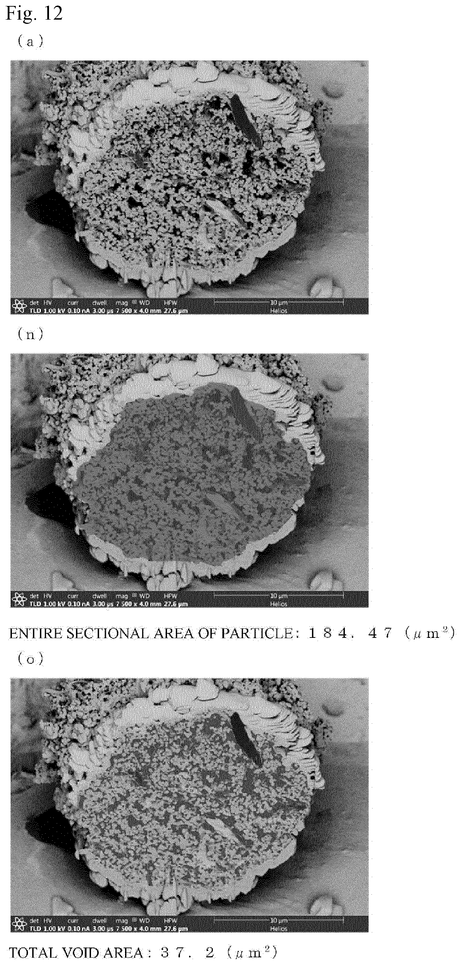

[Fig. 12] Figure (a) is a SEM image of a cross section of a particle obtained in Example 15; Figure (b) is a SEM image highlighting the entire cross section (the sectional area) of the particle used in the calculation of void ratio; and Figure (c) is a SEM image highlighting the voids in the cross section of the particle used in the calculation of void ratio.

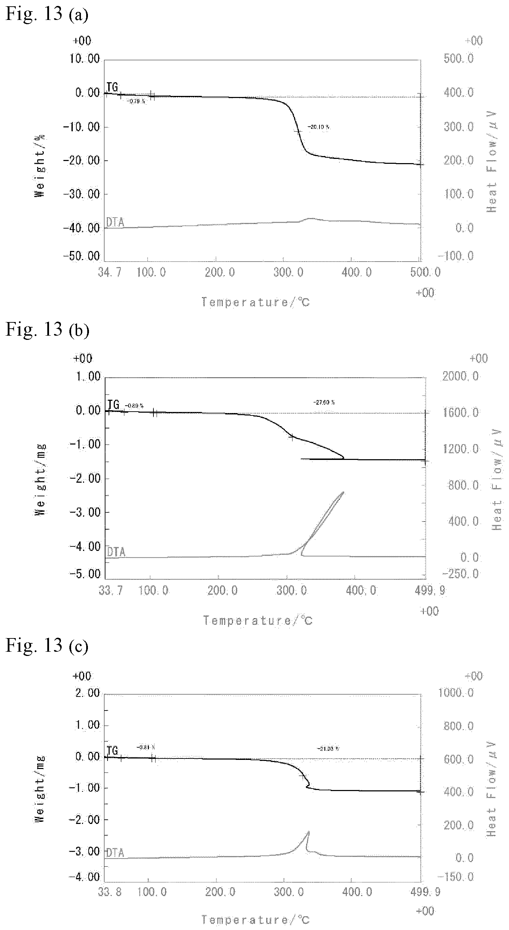

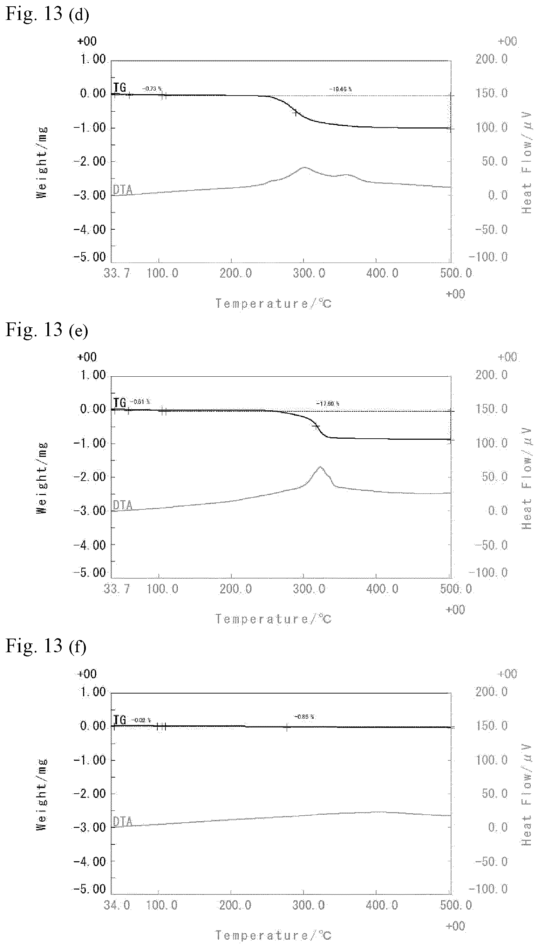

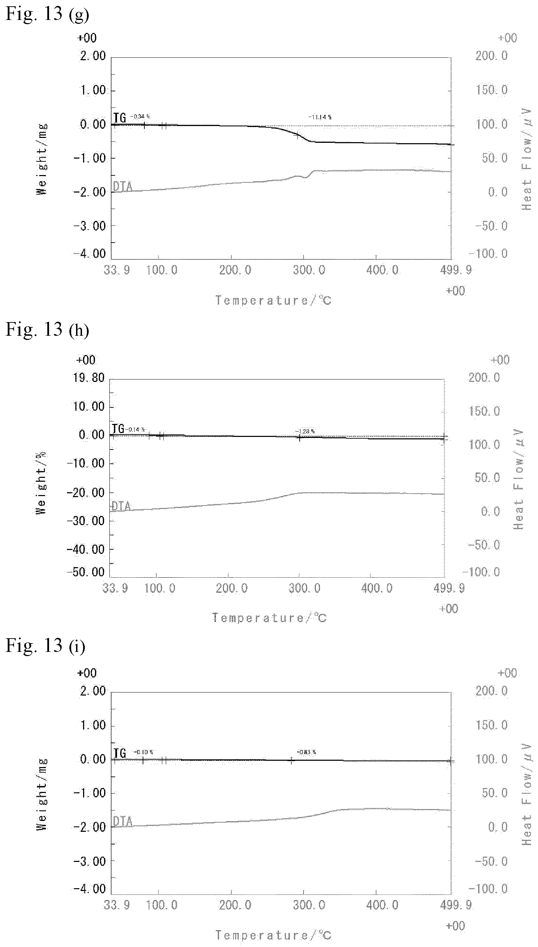

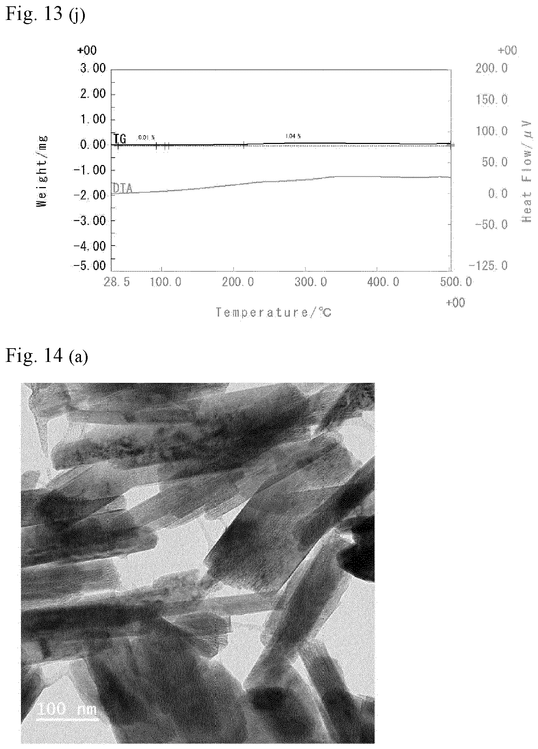

[Fig. 13] Figures (a) to (e) are the results of thermogravimetric-differential thermal analysis (TG-DTA) of particles obtained in Examples 9 and 12 to 15, respectively; Figures (f) to (j) are the results of TG-DTA of inorganic pigments used in the respective particles, specifically, Figure (f) is the result of TG-DTA of titanium oxide (MKR-1), Figure (g) is the result of TG-DTA of yellow iron oxide (LL-100HP), Figure (h) is the result of TG-DTA of red iron oxide (C33-8001), Figure (i) is the result of TG-DTA of red iron oxide (R-516HP), and Figure (j) is the result of TG-DTA of black iron oxide (BL-100HP).

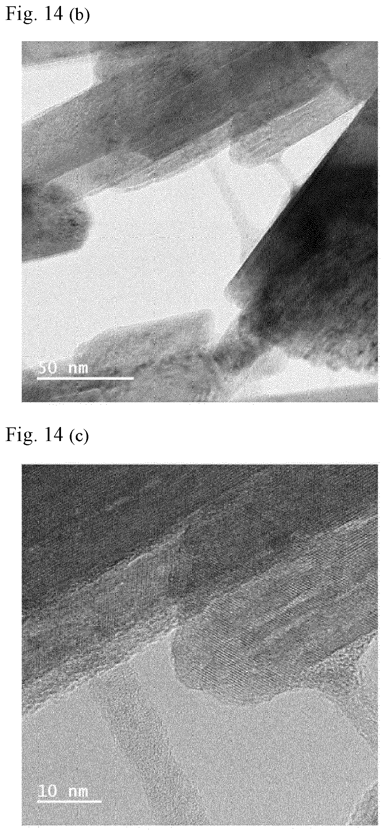

[Fig. 14] Figures (a) to (c) are transmission electron microscope (TEM) images at different magnifications of particles obtained in Example 12.

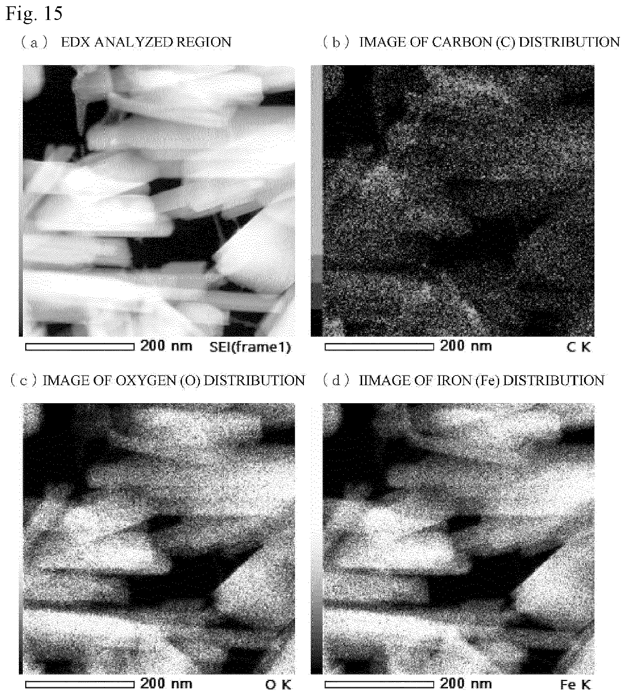

[Fig. 15] Figures (a) to (d) are an image of an analyzed region, and images of distribution of carbon (C), oxygen (O) and iron (Fe), respectively, obtained by energy dispersive X-ray analysis (TEM-EDX) of particles from Example 12.

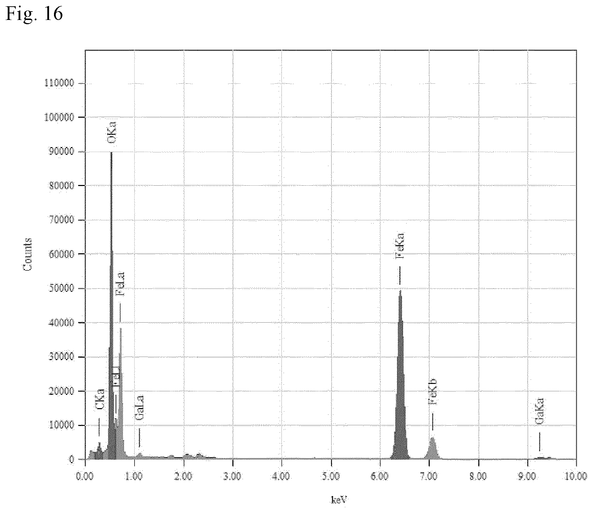

[Fig. 16] Figure 16 is an EDX spectrum obtained by energy dispersive X-ray analysis (TEM-EDX) of particles from Example 12.

Description of Embodiments

(Particles)

The present invention pertains to porous pigment particles containing cellulose or a cellulose derivative, and an inorganic pigment as main components. The term "porous" in the present invention means that the surface of pigment particles is uneven and has a plurality of pores. Specifically, the porous pigment particles of the present invention have grooved streaks (an uneven structure) that look like wrinkles or pleats when observed with respect to an enlarged image of the particles. The porous particles of the present invention may be characterized by having a void ratio in the predetermined range described later, or by having a pore surface area of 0.1 $m^2/g$ or more, preferably 1 $m^2/g$ or more, more preferably 5 $m^2/g$ or more, and 100 $m^2/g$ or less, preferably 50 $m^2/g$ or less, more preferably 30 $m^2/g$ or less in the pore size distribution measurement by a mercury intrusion method described later in Examples.

The particles of the present invention contain cellulose or a cellulose derivative as a main component. Examples of the celluloses and the cellulose derivatives used in the present invention include those derived from natural fibers such as wool, cotton, silk, hemp or pulp, those derived from regenerated fibers such as rayons, polynosic fibers, cupra fibers (Bemberg (registered trademark)) or lyocell fibers (Tencel (registered trademark)), and those produced by bacteria. Examples further include those derived from cellulose composite fibers containing cellulose fibers and synthetic fibers (for example, fibers of a polyolefin such as polyethylene or polypropylene).

The celluloses and the cellulose derivatives used in the present invention may be those derived from natural fibers, for example, those derived from plants such as wood, bamboos, hemp, jute, kenaf, cotton, beet and agricultural wastes. In particular, those derived from broad-leaved trees, coniferous trees or bamboos may be used. α-Cellulose derived from such a fibrous plant is preferably partially depolymerized with an acid and then purified. That is, it is preferable to use, for example, crystalline cellulose, more preferably microcrystalline cellulose obtained as described above. For example, such cellulose nanofibers may be commercially obtained from suppliers such as FUSHIMI Pharmaceutical Co., Ltd.

The celluloses and the cellulose derivatives used in the present invention may be cellulose nanofibers. The "cellulose nanofibers (CNF)" are fibers obtained by defibrating cellulose fibers to a nano-size level, and generally have a fiber width of about 4 to 200 nm and a fiber length of about 5 μm or more. Such cellulose nanofibers may be prepared by a known method or may be purchased from the market. For example, such nanofibers may be obtained from suppliers such as Daio Paper Corporation and Chuetsu Pulp & Paper Co., Ltd.

The particles of the present invention also contain an inorganic pigment as a main component. The inorganic pigments used in the present invention are inorganic pigments that can impart properties such as coloration properties, masking properties, hiding properties and smoothing properties to target products (for example, cosmetics). Typically, the inorganic pigments are color pigments for adjusting the color tone of products, white pigments for controlling masking properties and hiding properties, pearlescent pigments for imparting so-called pearly finish, or functional pigments having combined functions such as hiding properties and smoothing properties. Examples include color pigments such as rouge (red iron oxide), yellow iron oxide, black iron oxide, chromium oxide, ultramarine blue, Prussian blue, manganese purple and carbon black; white pigments such as titanium oxide (titanium dioxide), zinc oxide (Chinese white), aluminum oxide (alumina), zirconium oxide and barium sulfate; pearlescent pigments such as bismuth oxychloride, aluminum powder, red iron oxide-coated mica and titanium oxide-coated mica (titanated mica); and functional pigments such as boron nitride, synthetic fluorine phlogopite (synthetic mica), photochromic pigments and UV-absorbing mica. From the points of view of the combination with the cellulose or the cellulose derivative and the application of the particles to cosmetics, the inorganic pigment is preferably at least one selected from the group consisting of color pigments and white pigments, specifically, red iron oxide, yellow iron oxide, black iron oxide, chromium oxide, ultramarine blue, Prussian blue, manganese purple, titanium oxide, zinc oxide, aluminum oxide and zirconium oxide. A functional pigment, specifically, boron nitride is also preferable. These inorganic pigments may be obtained from suppliers as additives for pharmaceuticals, foods or cosmetics.

In the present invention, the phrase that the porous pigment particles "comprise cellulose or a cellulose derivative, and an inorganic pigment as main components" means that the ratio (by mass) of the cellulose or the cellulose derivative, and the inorganic pigment is more than 50 mass% of the particles. The ratio (by mass) of the cellulose or the cellulose derivative, and the inorganic pigment is preferably 60 mass% or more, more preferably 70 mass% or more, still more preferably 80 mass% or more, and particularly preferably 90 mass% or more. In the most preferred embodiment, the particles of the present invention consist solely of the cellulose or the cellulose derivative, and the inorganic pigment.

In the present invention, the cellulose or the cellulose derivative, and the inorganic pigment are blended in such amounts that the ratio (by mass) of the inorganic pigment is 1 to 20 parts by mass, preferably 2 to 10 parts by mass, and more preferably 3 to 5 parts by mass relative to 1 part by mass of the cellulose or the cellulose derivative.

In addition to the cellulose or the cellulose derivative, and the inorganic pigment, the particles may contain additional components, for example, inorganic powders of various sizes and shapes such as magnesium carbonate, calcium carbonate, aluminum silicate, barium silicate, calcium silicate, magnesium silicate, strontium silicate, metal tungstate salts, calcined calcium sulfate, calcium phosphate, fluoroapatite, hydroxyapatite, ceramic powders, metallic soaps (for example, zinc myristate, calcium palmitate, aluminum stearate), silica and fumed silica (ultrafine silicic anhydride particles), and powders obtained by hydrophobizing or hydrophilizing the above inorganic powders by treatment with various kinds of surface treatment agents such as silicones including hydrogen silicone and cyclic hydrogen silicone, and other silane or titanium coupling agents.

The particle size of the particles of the present invention may be selected appropriately in accordance with the desired use application of the particles. For example, the particle sizes are distributed in the range of 0.1 to 500 $\mu$m, preferably in the range of 1 to 200 $\mu$m, more preferably 2 to 100 $\mu$m, and particularly preferably in the range of 5 to 80 $\mu$m. The average particle size is, for example, in the range of 5 to 40 $\mu$m, and preferably in the range of 5 to 30 $\mu$m. In the present invention, the particle size means a value measured with a scattering-type particle size distribution analyzer, and the average particle size means the arithmetic mean diameter calculated from the grain size distribution that is obtained.

The void ratio of the particles of the present invention may be selected appropriately in accordance with the desired use application of the particles. For example, the void ratio is in the range of 5 to 50%, preferably in the range of 5 to 40%, more preferably in the range of 5 to 30%, and still more preferably in the range of 10 to 30%. In the present invention, the void ratio is a value measured with respect to a sectional image of a particle captured with a device such as a scanning electron microscope, and indicates the percentage of the area of voids (the total area of voids in the sectional image of the particle) relative to the sectional area (the area of the entire cross section in the sectional image of the particle) taken as 100. The average void ratio means the arithmetic average of the porosities obtained as described above. Specifically, the void ratio of the particles of the present invention may be calculated in accordance with Evaluation Example 5 described later. The above range of the void ratio of the particles of the present invention ensures that the particles are soft and exhibit excellent optical characteristics even after formulated into a foundation.

(Light scattering rate)

The particles of the present invention are characterized in that the light scattering rate indicated by the following equation (1) is in the range of 50 to 230%.

$$\text{Light scattering rate (\%)} = \frac{(\text{Reflection intensity at } 20° \text{ angle} \diagup \cos 20°) + (\text{Reflection intensity at } 70° \text{ angle} \diagup \cos 70°)}{2 \times (\text{Reflection intensity at } 5° \text{ angle} \diagup \cos 5°)} \times 100 \cdots (1)$$

In the equation (1), the reflection intensities at angles of 20°, 70° and 5° are determined in the following manner. Light is caused to be incident on the particles at an angle of -30° relative to the normal to the plane in contact with the particles taken as 0°, and the sensitivity of a light receiver is set to an arbitrary value (which is referred to as the sensitivity control value). Thereafter, light is caused to be incident at a certain angle, and the intensities are measured of reflected light at light receiver angles of 20°, 70° and 5°.

Here, the light scattering rate is calculated in accordance with the above equation (1) described in WO 2010/092890. When, for example, the incident angle of light is -30°, the calculation of the light scattering rate starts with setting the sensitivity of the light receiver to an arbitrary value (which is referred to as the sensitivity control value) when the sample is irradiated with light at an angle of -30° relative to the normal to the plane in contact with the sample taken as 0°, and the intensities are measured of reflected light at light receiver angles of 20°, 70° and 5°. Next, while maintaining the initial sensitivity control value, the sample is irradiated with light at an angle of -45° and the intensities are measured of reflected light at light receiver angles of 20°, 70° and 5°. The relative intensities are measured similarly at an incident angle of -60°. Lastly, the light scattering rate is calculated.

The above equation (1) means that when the light scattering rate is 100%, the graph representing the reflection intensities is circular and the incident light is uniformly diffused; when the light scattering rate exceeds 100%, the graph representing the reflection intensities is a horizontal ellipse; and when the light scattering rate is less than 100%, the graph is a vertical ellipse.

The particles of the present invention reflect light with more uniform intensities in spite of the fact that the surface thereof has a wrinkle-like or fold-like uneven structure. The particles of the present invention are characterized by having a light scattering rate in the range of 50 to 230%. In observation at light incident angles of -30°, -45° and -60°, the light scattering rate is preferably in the range of 50 to 230% at any one of the above incident angles, more preferably in the range of 50 to 230% at any two of the above incident angles, and still more preferably in the range of 50 to 230% at all of the above incident angles. The particles of the present invention more preferably have a light scattering rate in the range of 70 to 230%, and still more preferably in the range of 90 to 230%. Having such a light scattering rate means that the particles of the present invention can reflect light in all directions more uniformly and are expected to produce the defocus effect when the particles of the present invention are used as, for example, an additive for cosmetics.

In particular, in a measurement of distribution of the reflected light, it is preferable that a ratio of the maximum intensity of the reflected light in the vicinity of specular reflection (for example, within an angle of specular reflection $\pm10°$, preferably within an angle of specular reflection $\pm5°$) to the incident light, to the maximum intensity of the reflected light in the vicinity of the incident light (for example, within an angle of incident light $\pm10°$, preferably within an angle of incident light $\pm5°$) is in the range of 0.5:1 to 2:1, and preferably in the range of 0.5:1 to 1.8:1. When the intensity of specular reflection is in such a range, it can be expected to exhibit excellent optical characteristics, in particular, the defocus effect when the particles of the present invention are used as an additive for cosmetics (in particular, an additive for a foundation).

(Methods for producing particles)

The particles of the present invention may be produced by a method containing a step of obtaining a dispersion of cellulose or a cellulose derivative, and an inorganic pigment, and a step of spray-drying the dispersion obtained.

Examples and preferred embodiments of the cellulose or the cellulose derivative and of the inorganic pigment in the dispersion in the production method of the present invention are the same as described hereinabove. The dispersion may be prepared by any method, for example, by mixing together cellulose or a cellulose derivative, an inorganic pigment and a dispersion medium, and pulverizing the mixture. Alternatively, the dispersion may be obtained by mixing cellulose or a cellulose derivative (or an inorganic pigment) together with a dispersion medium, pulverizing the mixture to prepare a cellulose or cellulose derivative (or inorganic pigment) dispersion, then mixing the dispersion together with an inorganic pigment (or cellulose or a cellulose derivative) and a dispersion medium, and further pulverizing the resultant mixture. The dispersion medium is preferably an aqueous medium, more preferably water, a water-miscible organic solvent or a mixture thereof. Examples of the water-miscible organic solvents include C1-C4 alcohols such as methanol, ethanol, isopropyl alcohol and butanol, ketones such as acetone, nitriles such as acetonitrile, amides such as N-methylpyrrolidone, N-cyclohexylpyrrolidone, N,N-dimethylacetamide and N,N-dimethylformamide, lactones such as $\gamma$-butyrolactone, and ethers such as tetrahydrofuran. In the most preferred embodiment, the dispersion medium is water or a mixture of water and a C1-C4 alcohol.

The dispersion contains the inorganic pigment in an amount of 1 to 20 parts by mass, preferably 2 to 10 parts by mass, and more preferably 3 to 5 parts by mass with respect to 1 part by mass of the cellulose or the cellulose derivative. The concentration of the solids containing the cellulose or the cellulose derivative, and the inorganic pigment in the dispersion is not particularly limited as long as the dispersion may be used in the subsequent spray-drying step, but is, for example, 0.5 to 50 mass%, preferably 5 to 50 mass%, and more preferably 10 to 40 mass%.

The dispersion may be obtained in any manner without limitation while using an operation that is known to those skilled in the art. Typically, the dispersion is obtained by pulverization, preferably physical pulverization, of the cellulose or the cellulose derivative, and the inorganic pigment. The physical pulverization is performed by applying a physical external force to a mixture of the cellulose or the cellulose derivative, and/or the inorganic pigment, and the dispersion medium with use of a stirrer such as a magnetic stirrer or a stirring blade, a homogenizer such as POLYTRON homogenizer, an ultrasonic generator such as an ultrasonic crusher, or a pulverizer such as a wet pulverization device (for example, Star Burst; SUGINO MACHINE LIMITED). When the cellulose or the cellulose derivative, and/or the inorganic pigment is a sufficiently pulverized commercial product, the dispersion may be obtained without performing the pulverization treatment. That is, the production method of the present invention may use a commercially available cellulose dispersion (or a commercially available inorganic pigment dispersion), for example, a commercially available cellulose nanofiber dispersion, instead of performing the step of obtaining a cellulose or cellulose derivative (or inorganic pigment) dispersion.

The particles of the present invention are obtained by spray-drying the dispersion obtained. The spray-drying is performed using a known spray-drying device such as an atomizer, a spray dryer or a micro mist spray dryer. The spray-drying conditions are determined appropriately in accordance with factors such as the type of the dispersion medium in the dispersion, and the type or the concentration of the cellulose or the cellulose derivative. For example, the spray-drying may be performed at an inlet temperature of 150 to 300°C and an outlet temperature of 0 to 150°C.

(Cosmetics)

The porous pigment particles of the present invention are resistant to aggregation and are excellently dispersed in a base material to impart a color while improving the dullness problem. Thus, the porous pigment particles are suitably added to cosmetics or quasi-drugs that require coloration properties, masking properties and hiding properties. Further, the particles of the present invention contain cellulose and have a wrinkle-like or fold-like uneven structure on the surface thereof and also an appropriate amount of pores or voids inside the particles to offer soft and comfortable texture. In view of this, the particles of the present invention are suitably added to cosmetics that are directly applied to the skin. Further, the particles of the present invention have excellent optical characteristics (light scattering properties) in which incident light is uniformly scattered. Thus, the particles of the present invention are particularly suited for addition to cosmetics that are applied directly to the skin and are required to have such optical characteristics as the defocus effect. Examples of such cosmetics include skin care products such as lotions, gels and emulsions, toiletry products such as facial cleansing foams, facial cleansing powders and body washes, hair care products such as shampoos and conditioners, oral care products such as dentifrices, and makeup cosmetics such as foundations, powder foundations, liquid foundations, BB creams, concealers, lipsticks and sunscreens. In these products, the particles of the present invention may be used as an inorganic pigment or as a light-scattering agent for producing the defocus effect.

When added to cosmetics or quasi-drugs, the porous pigment particles of the present invention may be surface-treated with surface treatment agents usually used in cosmetics as long as the effects of the porous pigment particles are not impaired. Specifically, the surface treatment may be performed using one, or two or more kinds of the hydrophobizing agents described below.

(1) Treatment with silicones such as methylhydrogenepolysiloxane (KF99P manufactured by Shin-Etsu Chemical Co., Ltd.), methylhydrogenepolysiloxane/dimethylpolysiloxane copolymer (KF-9901 manufactured by Shin-Etsu Chemical Co., Ltd.), dimethylpolysiloxane, branched silicone (ethoxy functional) treatment agents (KF9908 and KF9909 manufactured by Shin-Etsu Chemical Co., Ltd.) and acrylic silicone treatment agent (KP574 manufactured by Shin-Etsu Chemical Co., Ltd.).
(2) Treatment with alkylalkoxysilane compounds such as octyltriethoxysilane (DYNASILAN OCTEO manufactured by EVONIK DEGUSSA), hexyltrimethoxysilane and octadecyltriethoxysilane.
(3) Treatment with fatty acids such as palmitic acid and stearic acid.
(4) Treatment with alkylated sugars such as dextrin palmitates in which part of the hydroxyl groups in dextrins are alkyl esterified; and treatment with metallic soaps such as alkali metal salts or alkaline earth metal salts of fatty acids.
(5) Treatment with amino acids such as N-lauroyl-L-lysine, glutamic acid and alkali salts thereof, and acylated amino acids.
(6) Treatment with fluorine agents such as perfluoroalkylphosphoric acid diethanolamine salts, perfluoroalkylphosphoric acids, perfluorohexylethyl triethoxysilane, perfluorooctylethyl triethoxysilane, and copolymers containing a C4-C6 perfluoroalkyl chain functional group.
(7) Treatment with quaternary ammonium salts such as distearyldimethylammonium chloride.

Surface treatment methods other than those described above may be used, with examples including higher alcohol treatment, lecithin treatment, hydrogenated lecithin treatment, amino acid treatment, collagen treatment, elastin treatment, ester treatment, wax treatment, surfactant treatment and moisturizing treatment.

The porous pigment particles of the present invention may also be used as an additive for cosmetic container (package) resins. Examples of such resins include:

- Synthetic resins such as polyethylene, polypropylene, polystyrene and polyethylene terephthalate, derivatives thereof, and composite materials thereof.
- Biodegradable resins.
- Composite materials of synthetic resins and biodegradable resins.

Examples of the biodegradable resins include aliphatic polyesters such as polylactic acid, polyvinyl alcohol, poly(butylene adipate/terephthalate), polybutylene succinate, polyhydroxyalkanoates, (polylactic acid/polybutylene succinate) block copolymer, polycaprolactone, poly(caprolactone/butylene succinate), poly(butylene succinate/adipate), poly(butylene succinate/carbonate), poly(ethylene terephthalate/succinate), poly(tetramethylene adipate/terephthalate), polyethylene succinate and polyglycolic acid; modified starches; casein plastics; and celluloses. The biodegradable resins may be used singly, or two or more may be used in combination.

Where necessary, the porous pigment particles of the present invention may be used in combination with various additives which may be used as additives for cosmetics and quasi-drugs.

Exemplary additive components such as physiologically active substances and functional substances added to dermatological preparations such as cosmetics and quasi-drugs are oily bases, moisturizers, touch improvers, surfactants, polymers, thickening or gelling agents, solvents, propellants, antioxidants, reducing agents, oxidizing agents, antiseptic agents, antibacterial agents, bactericidal agents, chelating agents, pH adjusting agents, acids, alkalis, powders, inorganic salts, ultraviolet absorbers, whitening agents, vitamins and derivatives thereof, hair-growth agents, blood circulation promoters, stimulants, hormones, anti-wrinkle agents, anti-aging agents, tightening agents, cooling sensation agents, warming sensation agents, wound healing accelerators, irritation mitigating agents, analgesics, cell activators, plant/animal/microbial extracts, antipruritic agents, keratin exfoliating/dissolving agents, antiperspirants, algefacients, astringents, enzymes, nucleic acids, fragrances, colorants, coloring agents, dyes, pigments, antiphlogistics, anti-inflammatory agents, anti-asthmatic agents, anti-chronic obstructive pulmonary disease agents, anti-allergic agents, immunomodulators, anti-infective agents and antifungal agents.

Examples of these additive components will be described below. Preferred examples of the oily bases include higher (polyhydric) alcohols such as cetanol, myristyl alcohol, oleyl alcohol, lauryl alcohol, cetostearyl alcohol, stearyl alcohol, arachidyl alcohol, behenyl alcohol, jojoba alcohol, chimyl alcohol, selachyl alcohol, batyl alcohol, hexyldecanol, isostearyl alcohol, 2-octyldodecanol and dimer diol; aralkyl alcohols such as benzyl alcohol, and derivatives thereof; higher fatty acids such as lauric acid, myristic acid, palmitic acid, stearic acid, isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteisoheneicosanoic acid, long-chain branched fatty acids, dimer acids and hydrogenated dimer acids, metallic soaps thereof such as aluminum salts, calcium salts, magnesium salts, zinc salts, potassium salts and sodium salts, and nitrogen-containing derivatives thereof such as amides; hydrocarbons such as liquid paraffin (mineral oils), heavy liquid isoparaffin, light liquid isoparaffin, $\alpha$-olefin oligomers, polyisobutene, hydrogenated polyisobutene, polybutene, squalane, olive-derived squalane, squalene, vaseline and solid paraffin; waxes such as candelilla wax, carnauba wax, rice wax, wood wax, beeswax, montan wax, ozokerite, ceresin, paraffin wax, microcrystalline wax, petrolatum, Fischer-Tropsch wax, polyethylene wax and ethylene-propylene copolymer; vegetable oils and fats such as coconut oil, palm oil, palm kernel oil, safflower oil, olive oil, castor oil, avocado oil, sesame oil, tea oil, evening primrose oil, wheat germ oil, macadamia nut oil, hazelnut oil, kukui nut oil, rose hip oil, meadowfoam oil, persic oil, tea tree oil, peppermint oil, corn oil, rapeseed oil, sunflower oil, wheat germ oil, linseed oil, cottonseed oil, soybean oil, peanut oil, rice bran oil, cacao butter, shea butter, hydrogenated coconut oil, hydrogenated castor oil, jojoba oil and hydrogenated jojoba oil; animal oils and fats such as beef tallow, milk fat, horse fat, egg yolk oil, mink oil and turtle oil; animal waxes such as whale wax, lanolin and orange roughy oil; lanolins such as liquid lanolin, hydrogenated lanolin, adsorption refined lanolin, lanolin acetate, liquid lanolin acetate, hydroxylanolin, polyoxyethylenelanolin, lanolin fatty acid, hard lanolin fatty acid, lanolin alcohol, lanolin alcohol acetate and (cetyl-lanolyl) acetate; phospholipids such as lecithin, phosphatidylcholine, phosphatidylethanolamine, phosphatidylserine, phosphatidylglycerol, phosphatidylinositol, sphingophospholipids including sphingomyelin, phosphatidic acid and lysolecithin; phospholipid derivatives such as hydrogenated soybean phospholipids, partially hydrogenated soybean phospholipids, hydrogenated egg yolk phospholipids and partially hydrogenated egg yolk phospholipids; sterols such as cholesterol, dihydrocholesterol, lanosterol, dihydrolanosterol, phytosterol and cholic acid; sapogenins; saponins; sterol esters such as cholesteryl acetate, cholesteryl nonanoate, cholesteryl stearate, cholesteryl isostearate, cholesteryl oleate, di(cholesteryl/behenyl/octyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, acylsarcosine alkyl esters including N-lauroylsarcosine isopropyl, cholesteryl 12-hydroxystearate, macadamia nut oil fatty acid cholesteryl ester, phytosteryl macadamia nut oil fatty acid ester, phytosteryl isostearate, soft lanolin fatty acid cholesteryl ester, hard lanolin fatty acid cholesteryl ester, long-chain branched fatty acid cholesteryl ester and long-chain $\alpha$-hydroxy fatty acid cholesteryl ester; lipid complexes such as phospholipid-cholesterol complex and phospholipid-phytosterol complex; monoalcohol carboxylic acid esters such as octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, octyldodecyl lanolin fatty acid ester, hexyldecyl dimethyloctanoate, octyldodecyl erucate, hydrogenated castor oil isostearate, ethyl oleate, avocado oil fatty acid ethyl ester, isopropyl myristate, isopropyl palmitate, octyl palmitate, isopropyl isostearate, lanolin fatty acid isopropyl ester, diethyl sebacate, diisopropyl sebacate, dioctyl sebacate, diisopropyl adipate, dibutyloctyl sebacate, diisobutyl adipate, dioctyl succinate and triethyl citrate; oxyacid esters such as cetyl lactate, diisostearyl malate and hydrogenated castor oil monoisostearate; polyhydric alcohol fatty acid esters such as glyceryl trioctanoate, glyceryl trioleate, glyceryl triisostearate, glyceryl diisostearate, glyceryl tri(caprylate/caprate), glyceryl tri(caprylate/caprate/myristate/stearate), hydrogenated rosin triglyceride (hydrogenated ester gum), rosin triglyceride (ester gum), glyceryl behenate eicosanedioate, trimethylolpropane trioctanoate, trimethylolpropane triisostearate, neopentyl glycol dioctanoate, neopentyl glycol dicaprate, 2-butyl-2-ethyl-1,3-propane diol dioctanoate, propylene glycol dioleate, pentaerythrityl tetraoctanoate, hydrogenated rosin pentaerythrityl, ditrimethylolpropane triethylhexanoate, ditrimethylolpropane (isostearate/sebacate), pentaerythrityl triethylhexanoate, dipentaerythrityl (hydroxystearate/stearate/rosinate), diglyceryl diisostearate, polyglyceryl tetraisostearate, polyglyceryl-10 nonaisostearate, polyglyceryl-8 deca(erucate/isostearate/ricinoleate), diglyceryl (hexyldecanoic acid/sebacic acid) oligo ester, glycol distearate (ethylene glycol distearate), 3-methyl-1,5-pentanediol dineopentanoate and 2,4-diethyl-1,5-pentanediol dineopentanoate; dimer acid or dimer diol derivatives such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosin condensate, hydrogenated castor oil dimer dilinoleate and hydroxyalkyl dimer dilinoleyl ether; fatty acid alkanolamides such as coconut fatty acid monoethanolamide (cocamide MEA), coconut fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (paltamide MEA), palmitic acid diethanolamide (paltamide DEA) and coconut fatty acid methylethanolamide (cocamide methyl MEA); silicones such as dimethicone(dimethylpolysiloxane), highly polymerized dimethicone (highly polymerized dimethylpolysiloxane), cyclomethicone (cyclic dimethylsiloxane, decamethylcyclopentasiloxane), phenyltrimethicone, diphenyldimethicone, phenyldimethicone, stearoxypropyldimethylamine, (aminoethyl aminopropyl methicone/dimethicone) copolymer, dimethiconol, dimethiconol crosspolymer, silicone resin, silicone rubber, amino-modified silicones including aminopropyldimethicone and amodimethicone, cation-modified silicones, polyether-modified silicones including dimethicone copolyol, polyglycerin-modified silicone, sugar-modified silicone, carboxylic acid-modified silicone, phosphoric acid-modified silicone, sulfuric acid-modified silicone, alkyl-modified silicone, fatty acid-modified silicone, alkyl ether-modified silicone, amino acid-modified silicone, peptide-modified silicone, fluorine-modified silicone, cation-modified and polyether-modified silicone, amino-modified and polyether-modified silicone, alkyl-modified and polyether-modified silicone and polysiloxane-oxyalkylene copolymer; and fluorine-based oil agents such as perfluorodecane, perfluorooctane and perfluoropolyether.

Preferred examples of the moisturizers and the touch improvers include polyols and polymers thereof such as glycerin, 1,3-butylene glycol, propylene glycol, 3-methyl-1,3-butanediol, 1,3-propanediol, 2-methyl-1,3-propanediol, trimethylolpropane, pentaerythritol, hexylene glycol, diglycerin, polyglycerin, diethylene glycol, polyethylene glycol, dipropylene glycol, polypropylene glycol and ethylene glycol-propylene glycol copolymer; glycol alkyl ethers such as diethylene glycol monoethyl ether (ethoxydiglycol), ethylene glycol monoethyl ether, ethylene glycol monobutyl ether and diethylene glycol dibutyl ether; water-soluble esters such as polyglyceryl-10 (eicosanedioate/tetradecanedioate) and polyglyceryl-10 tetradecanedioate; sugar alcohols such as sorbitol, xylitol, erythritol, mannitol and maltitol; sugars and derivatives thereof such as glucose, fructose, galactose, mannose, threose, xylose, arabinose, fucose, ribose, deoxyribose, maltose, trehalose, lactose, raffinose, gluconic acid, glucuronic acid, cyclodextrins ($\alpha$-, $\beta$- and $\gamma$-cyclodextrins, and modified cyclodextrins including maltosylated cyclodextrin and hydroxyalkylated cyclodextrin), $\beta$-glucan, chitin, chitosan, heparin and derivatives thereof, pectin, arabinogalactan, dextrin, dextran, glycogen, ethyl glucoside, and glucosylethyl methacrylate polymers or copolymers; hyaluronic acid, sodium hyaluronate; sodium chondroitin sulfate; mucoitinsulfuric acid, charoninsulfuric acid, keratosulfuric acid, dermatansulfuric acid; Tremella fuciformis extract, Tremella fuciformis polysaccharides; fucoidan; Polianthes tuberosa polysaccharides or naturally occurring polysaccharides; organic acids such as citric acid, tartaric acid and lactic acid, and salts thereof; urea and derivatives thereof; 2-pyrrolidone-5-carboxylic acid and salts thereof such as sodium salt; amino acids and salts thereof such as betaine (trimethylglycine), proline, hydroxyproline, arginine, lysine, serine, glycine, alanine, phenylalanine, tyrosine, $\beta$-alanine, threonine, glutamic acid, glutamine, asparagine, aspartic acid, cystine, cysteine, methionine, leucine, isoleucine, valine, tryptophan, histidine and taurine; protein peptides and derivatives thereof such as collagen, fish-derived collagen, atelocollagen, gelatin, elastin, collagen degradation peptide, hydrolyzed collagen, hydroxypropylammonium chloride hydrolyzed collagen, elastin degradation peptide, keratin degradation peptide, hydrolyzed keratin, conchiolin degradation peptide, hydrolyzed conchiolin, silk protein degradation peptide, hydrolyzed silk, sodium lauroyl hydrolyzed silk, soybean protein degradation peptide, wheat protein degradation peptide, hydrolyzed wheat protein, casein degradation peptide and acylated peptide; acylated peptides such as palmitoyl oligopeptide, palmitoyl pentapeptide and palmitoyl tetrapeptide; silylated peptides; lactic acid bacteria culture fluid, yeast extract, egg shell membrane protein, bovine submaxillary mucin, hypotaurine, sesame lignan glycosides, glutathione, albumin, milk serum; choline chloride, phosphorylcholine; animal and plant extract components such as placenta extract, elastin, collagen, aloe extract, hamamelis water, loofah water, chamomile extract, licorice extract, comfrey extract, silk extract, Rosa roxburghii fruit extract, Achillea Millefolium extract, eucalyptus extract and melilot extract, and ceramides such as natural ceramides (types 1, 2, 3, 4, 5 and 6), hydroxyceramides, pseudoceramides, glycosphingolipids, and ceramide- and ceramide saccharide-containing extracts.

Preferred examples of the surfactants include anionic surfactants, nonionic surfactants, cationic surfactants, amphoteric surfactants and polymer surfactants. Preferred surfactants will be exemplified below. Preferred examples of the anionic surfactants include fatty acid salts such as potassium laurate and potassium myristate; alkyl sulfuric acid ester salts such as sodium lauryl sulfate, triethanolamine lauryl sulfate and ammonium lauryl sulfate; polyoxyethylenealkyl sulfate salts such as sodium laureth sulfate and triethanolamine laureth sulfate; acyl N-methylamino acid salts such as sodium methyl cocoyl taurate, potassium methyl cocoyl taurate, sodium methyl lauroyl taurate, sodium methyl myristoyl taurate, sodium methyl lauroyl alanine, sodium lauroyl sarcosinate, triethanolamine lauroyl sarcosinate and sodium methyl alanine lauroyl glutamate; acyl amino acid salts such as sodium cocoyl glutamate, triethanolamine cocoyl glutamate, sodium lauroyl glutamate, sodium myristoyl glutamate, sodium stearoyl glutamate, ditriethanolamine palmitoyl aspartate and triethanolamine cocoyl alaninate; polyoxyethylene alkyl ether acetate salts such as sodium laureth acetate; succinic acid ester salts such as sodium lauroyl monoethanolamide succinate; fatty acid alkanolamide ether carboxylic acid salts; acyl lactate salts; polyoxyethylene aliphatic amine sulfate salts; fatty acid alkanolamide sulfate salts; fatty acid glyceride sulfate salts such as sodium hydrogenated coconut fatty acid glycerin sulfate; alkylbenzene polyoxyethylene sulfate salts; olefin sulfonate salts such as sodium α-olefin sulfonates; alkyl sulfosuccinate salts such as disodium lauryl sulfosuccinate and sodium dioctyl sulfosuccinate; alkyl ether sulfosuccinate salts such as disodium laureth sulfosuccinate, sodium monolauroyl monoethanolamide polyoxyethylene sulfosuccinate and sodium lauryl polypropylene glycol sulfosuccinate; alkylbenzene sulfonate salts such as sodium tetradecylbenzene sulfonate and triethanolamine tetradecylbenzene sulfonate; alkylnaphthalene sulfonate salts; alkane sulfonate salts; α-sulfo fatty acid methyl ester salts; acyl isethionate salts; alkyl glycidyl ether sulfonate salts; alkyl sulfoacetate salts; alkyl ether phosphoric acid ester salts such as sodium laureth phosphate, sodium dilaureth phosphate, sodium trilaureth phosphate and sodium monooleth phosphate; alkyl phosphoric acid ester salts such as potassium lauryl phosphate; sodium caseinate; alkyl aryl ether phosphate salts; fatty acid amide ether phosphate salts; phospholipids such as phosphatidyl glycerol, phosphatidyl inositol and phosphatidic acid; and silicone-based anionic surfactants such as carboxylic acid-modified silicones, phosphoric acid-modified silicones and sulfuric acid-modified silicones. Preferred examples of the nonionic surfactants include polyoxyethylene alkyl ethers with various numbers of oxyethylene units such as laureths (polyoxyethylene lauryl ethers), ceteths (polyoxyethylene cetyl ethers), steareths (polyoxyethylene stearyl ethers), beheneths (polyoxyethylene behenyl ethers), isosteareths (polyoxyethylene isostearyl ethers) and octyldodeceths (polyoxyethylene octyldodecyl ethers); polyoxyethylene alkylphenyl ethers; castor oil and hydrogenated castor oil derivatives such as polyoxyethylene hydrogenated castor oil, polyoxyethylene castor oil, polyoxyethylene hydrogenated castor oil monoisostearate, polyoxyethylene hydrogenated castor oil triisostearate, polyoxyethylene hydrogenated castor oil monopyroglutamate monoisostearate diester and polyoxyethylene hydrogenated castor oil maleic acid; polyoxyethylene phytosterols; polyoxyethylene cholesterols; polyoxyethylene cholestanols; polyoxyethylene lanolins; polyoxyethylene hydrogenated lanolins; polyoxyethylene-polyoxypropylene alkyl ethers such as polyoxyethylene-polyoxypropylene cetyl ether, polyoxyethylene-polyoxypropylene 2-decyl tetradecyl ether, polyoxyethylene-polyoxypropylene monobutyl ether, polyoxyethylene-polyoxypropylene hydrogenated lanolin and polyoxyethylene-polyoxypropylene glycerin ether; polyoxyethylene-polyoxypropylene glycols; (poly)glycerin polyoxypropylene glycols such as PPG-9 diglyceryl; glycerin fatty acid partial esters such as glyceryl stearate, glyceryl isostearate, glyceryl palmitate, glyceryl myristate, glyceryl oleate, coconut fatty acid glyceride, monocottonseed oil fatty acid glyceride, glycerin monoerucate, glycerin sesquioleate, glycerin α,α'-oleate pyroglutamate and glycerin monostearate malic acid; polyglycerin fatty acid esters such as polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate, polyglyceryl-6 distearate, polyglyceryl-10 distearate, polyglyceryl-2 tristearate, polyglyceryl-10 decastearate, polyglyceryl-2 isostearate, polyglyceryl-3 isostearate, polyglyceryl-4 isostearate, polyglyceryl-5 isostearate, polyglyceryl-6 isostearate, polyglyceryl-8 isostearate, polyglyceryl-10 isostearate, polyglyceryl-2 diisostearate (diglyceryl diisostearate), polyglyceryl-3 diisostearate (triglyceryl diisostearate), polyglyceryl-10 diisostearate (decaglyceryl diisostearate), polyglyceryl-2 triisostearate, polyglyceryl-2 tetraisostearate, polyglyceryl-10 decaisostearate, polyglyceryl-2 oleate, polyglyceryl-3 oleate, polyglyceryl-4 oleate, polyglyceryl-5 oleate, polyglyceryl-6 oleate, polyglyceryl-8 oleate, polyglyceryl-10 oleate, polyglyceryl-6 dioleate, polyglyceryl-2 trioleate and polyglyceryl-10 decaoleate; ethylene glycol monofatty acid esters such as ethylene glycol monostearate; propylene glycol monofatty acid esters such as propylene glycol monostearate; pentaerythritol partial fatty acid esters; sorbitol partial fatty acid esters; maltitol partial fatty acid esters; maltitol ethers; sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate and diglycerol sorbitan tetra-2-ethylhexylate; sugar derivative partial esters such as sucrose fatty acid esters, methyl glucoside fatty acid esters and trehalose undecylenate; alkyl glucosides such as caprylyl glucoside; alkyl polyglycosides; lanolin alcohols; hydrogenated lanolins; polyoxyethylene fatty acid mono- and diesters such as polyoxyethylene distearate, polyethylene glycol diisostearate, polyoxyethylene monooleate and polyoxyethylene dioleate; polyoxyethylene-propylene glycol fatty acid esters; polyoxyethylene glycerin fatty acid esters such as polyoxyethylene glycerin monostearate, polyoxyethylene glycerin monoisostearate, polyoxyethylene glycerin triisostearate and polyoxyethylene monooleate; polyoxyethylene sorbitan fatty acid esters such as polyoxyethylene sorbitan monooleate, polyoxyethylene sorbitan monostearate, polyoxyethylene sorbitan monooleate and polyoxyethylene sorbitan tetraoleate; polyoxyethylene sorbitol fatty acid esters such as polyoxyethylene sorbitol monolaurate, polyoxyethylene sorbitol monooleate, polyoxyethylene sorbitol pentaoleate and polyoxyethylene sorbitol monostearate; polyoxyethylene methylglucoside fatty acid esters; polyoxyethylene alkyl ether fatty acid esters; polyoxyethylene animal and vegetable oils and fats such as polyoxyethylene sorbitol beeswax; alkyl glyceryl ethers such as isostearyl glyceryl ether, chimyl alcohol, selachyl alcohol and batyl alcohol; polyhydric alcohol alkyl ethers; polyoxyethylene alkylamines; tetrapolyoxyethylene-tetrapolyoxypropylene-ethylenediamine condensates; natural surfactants such as saponin and sophorolipid; polyoxyethylene fatty acid amides; fatty acid alkanolamides such as coconut fatty acid monoethanolamide (cocamide MEA), coconut fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (paltamide MEA), palmitic acid diethanolamide (paltamide DEA) and coconut fatty acid methylethanolamide (cocamide methyl MEA); alkyldimethylamine oxides such as lauramine oxide, cocamine oxide, stearamine oxide and behenamine oxide; alkylethoxydimethylamine oxides; polyoxyethylene alkyl mercaptans; and silicone-based nonionic surfactants such as polyether-modified silicones including dimethicone copolyol, polysiloxane-oxyalkylene copolymers, polyglycerin-modified silicones and sugar-modified silicones. Preferred examples of the cationic surfactants include alkyltrimethylammonium chlorides such as behentrimonium chloride, steartrimonium chloride, cetrimonium chloride and lauryltrimonium chloride; alkyltrimethylammonium bromides such as steartrimonium bromide; dialkyldimethylammonium chlorides such as distearyldimonium chloride and dicocodimonium chloride; fatty acid amide amines and salts thereof such as stearamidopropyl dimethylamine and stearamidoethyl diethylamine; alkyl ether amines such as stearoxypropyl dimethylamine, and salts thereof and quaternary salts thereof; fatty acid amide type quaternary ammonium salts such as long-chain branched fatty acid (12 to 31) aminopropylethyl dimethylammonium ethylsulfates and lanolin fatty acid aminopropylethyl dimethylammonium ethylsulfates; polyoxyethylene alkylamines, and salts thereof and quaternary salts thereof; alkylamine salts; fatty acid amide guanidinium salts; alkyl ether ammonium salts; alkyl trialkylene glycol ammonium salts; benzalkonium salts; benzethonium salts; pyridinium salts such as cetylpyridinium chloride; imidazolinium salts; alkylisoquinolinium salts; dialkylmorpholinium salts; polyamine fatty acid derivatives; and silicone-based cationic surfactants such as amino-modified silicones including aminopropyl dimethicone and amodimethicone, cation-modified silicones, cation-modified and polyether-modified silicones, and amino-modified and polyether-modified silicones. Preferred examples of the amphoteric surfactants include N-alkyl-N,N-dimethylamino acid betaines such as lauryl betaine (betaine lauryl dimethylaminoacetate); fatty acid amide alkyl-N,N-dimethylamino acid betaines such as cocamidopropyl betaine and lauramidopropyl betaine; imidazoline type betaines such as sodium cocoamphoacetate and sodium lauroamphoacetate; alkylsulfobetaines such as alkyldimethyltaurines; sulfate type betaines such as alkyldimethylaminoethanol sulfate esters; phosphate type betaines such as alkyldimethylaminoethanol phosphate esters; phospholipids such as phosphatidylcholine, phosphatidylethanolamine, phosphatidylserine, sphingophospholipids including sphingomyelin, lysolecithin, hydrogenated soybean phospholipid, partially hydrogenated soybean phospholipid, hydrogenated egg yolk phospholipid, partially hydrogenated egg yolk phospholipid and hydroxylated lecithin; and silicone-based amphoteric surfactants. Preferred examples of the polymer surfactants include polyvinyl alcohol, sodium alginate, starch derivatives, tragacanth gum, acrylic acid-methacrylic acid alkyl copolymers; and silicone-based surfactants.

Preferred examples of the polymers, the thickening agents and the gelling agents include guar gum, locust bean gum, quince seed, carrageenan, galactan, gum arabic, tara gum, tamarind, furcelleran, karaya gum, sunset hibiscus, cara gum, tragacanth gum, pectin, pectic acid and salts such as sodium salt, alginic acid and salts such as sodium salt, mannan; starches such as rice, corn, potato and wheat; xanthan gum, dextran, succinoglucan, curdlan, hyaluronic acid and salts thereof, xanthan gum, pullulan, gellan gum, chitin, chitosan, agar, brown algae extract, chondroitin sulfate salt, casein, collagen, gelatin, albumin; celluloses and derivatives thereof such as methyl cellulose, ethyl cellulose, hydroxyethyl cellulose, hydroxypropyl cellulose, hydroxypropylmethyl cellulose, carboxymethyl cellulose and salts thereof including sodium salt, methylhydroxypropyl cellulose, sodium cellulose sulfate, cellulose dialkyldimethylammonium sulfates, crystalline cellulose and cellulose powder; starch derivatives such as starch-based polymers including soluble starch, carboxymethyl starch, methylhydroxypropyl starch and methyl starch, starch hydroxypropyltrimonium chloride and aluminum cornstarch octenylsuccinate; alginic acid derivatives such as sodium alginate and propylene glycol alginate ester; polyvinylpyrrolidone (PVP), polyvinyl alcohol (PVA), vinylpyrrolidone-vinyl alcohol copolymer, poly(vinyl methyl ether); polyethylene glycol, polypropylene glycol, polyoxyethylene-polyoxypropylene copolymer; amphoteric methacrylic acid ester copolymers such as (methacryloyloxyethyl carboxybetaine/alkyl methacrylate) copolymers and (acrylates/stearyl acrylate/ethylamine oxide methacrylate) copolymers; (dimethicone/vinyl dimethicone) crosspolymers, (alkyl acrylate/diacetone acrylamide) copolymers, (alkyl acrylate/diacetone acrylamide) copolymer AMP; polyvinyl acetate partially saponified products, maleic acid copolymers; vinyl pyrrolidone-dialkylaminoalkyl methacrylate copolymers; acrylic resin alkanolamines; polyesters, water-dispersible polyesters; polyacrylamides; polyacrylic acid ester copolymers such as polyethyl acrylate, carboxyvinyl polymers, polyacrylic acid and salts thereof such as sodium salt, acrylic acid-methacrylic acid ester copolymers; acrylic acid-methacrylic acid alkyl copolymers; cationized celluloses such as polyquaternium-10, diallyldimethylammonium chloride-acrylamide copolymers such as polyquaternium-7, acrylic acid-diallyldimethylammonium chloride copolymers such as polyquaternium-22, acrylic acid-diallyldimethylammonium chloride-acrylamide copolymers such as polyquaternium-39, acrylic acid-cationized methacrylic acid ester copolymers, acrylic acid-cationized methacrylamide copolymers, acrylic acid-methyl acrylate-methacrylamidopropyltrimethyl ammonium chloride copolymers such as polyquaternium-47, choline methacrylate ester chloride polymers; cationized oligosaccharides, cationized polysaccharides such as cationized dextran and guar hydroxypropyltrimonium chloride; polyethyleneimine; cation polymers; 2-methacryloyloxyethyl phosphorylcholine polymers such as polyquaternium-51, and copolymers such as with butyl methacrylate; polymer emulsions such as acrylic resin emulsions, polyethyl acrylate emulsions, polyacrylic alkyl ester emulsions, polyvinyl acetate resin emulsions, natural rubber latexes and synthetic latexes; nitrocelluloses; polyurethanes and various kinds of copolymers; various kinds of silicones; various kinds of silicone-based copolymers such as acrylic-silicone graft copolymers; various kinds of fluorine-based polymers; 12-hydroxystearic acid and salts thereof; dextrin fatty acid esters such as dextrin palmitate and dextrin myristate; silicic anhydride, fumed silica (ultrafine silicic anhydride particles), magnesium aluminum silicate, sodium magnesium silicate, metallic soaps, metal dialkyl phosphate salts, bentonite, hectorite, organic-modified clay minerals, sucrose fatty acid esters and fructooligosaccharide fatty acid esters. Among the above examples, celluloses and derivatives thereof, alginic acid and salts thereof, polyvinyl alcohol, hyaluronic acid and salts thereof, and collagens are preferable.

Preferred examples of the solvents and the propellants include lower alcohols such as ethanol, 2-propanol (isopropyl alcohol), butanol and isobutyl alcohol; glycols such as propylene glycol, 1,3-butylene glycol, diethylene glycol, dipropylene glycol and isopentyl diol; glycol ethers such as diethylene glycol monoethyl ether (ethoxydiglycol), ethylene glycol monoethyl ether, ethylene glycol monobutyl ether, triethylene glycol monoethyl ether, diethylene glycol diethyl ether, diethylene glycol dibutyl ether, propylene glycol monoethyl ether and dipropylene glycol monoethyl ether; glycol ether esters such as ethylene glycol monoethyl ether acetate, diethylene glycol monoethyl ether acetate and propylene glycol monoethyl ether acetate; glycol esters such as diethoxyethyl succinate and ethylene glycol disuccinate; benzyl alcohol, benzyloxyethanol, propylene carbonate, dialkyl carbonates, acetone, ethyl acetate, N-methylpyrrolidone; toluene; and such propellants as fluorocarbon, next-generation Freon; LPG, dimethyl ether and carbon dioxide.

Preferred examples of the antioxidants include tocopherol derivatives such as tocopherol (vitamin E) and tocopherol acetate; BHT, BHA; gallic acid derivatives such as propyl gallate; vitamin C (ascorbic acid) and/or derivatives thereof; erythorbic acid and derivatives thereof; sulfite salts such as sodium sulfite; hydrogen sulfite salts such as sodium hydrogen sulfite; thiosulfate salts such as sodium thiosulfate; metabisulfite salts; thiotaurine, hypotaurine; thioglycerol, thiourea, thioglycolic acid and cysteine hydrochloride salt.

Preferred examples of the reducing agents include thioglycolic acid, cysteine and cysteamine.

Preferred examples of the oxidizing agents include hydrogen peroxide solution, ammonium persulfate, sodium bromate and percarbonic acid.

Preferred examples of the antiseptic agents, the antibacterial agents and the bactericidal agents include hydroxybenzoic acid, and salts thereof and esters thereof such as methylparaben, ethylparaben, propylparaben and butylparaben; salicylic acid; sodium benzoate; phenoxyethanol; isothiazolinone derivatives such as methylchloroisothiazolinone and methylisothiazolinone; imidazolinium urea; dehydroacetic acid and salts thereof; phenols; halogenated bisphenols such as triclosan, acid amides, quaternary ammonium salts; trichlorocarbanide, zinc pyrithione, benzalkonium chloride, benzethonium chloride, sorbic acid, chlorhexidine, chlorhexidine gluconate, halocarban, hexachlorophene, hinokitiol; other phenols such as phenol, isopropylphenol, cresol, thymol, para-chlorophenol, phenylphenol and sodium phenylphenol; phenylethyl alcohol, photosensitizers, antibacterial zeolites and silver ions.

Preferred examples of the chelating agents include edetate salts (ethylenediaminetetraacetate salts) such as EDTA, EDTA2Na, EDTA3Na and EDTA4Na; hydroxyethylethylenediaminetriacetate salts such as HEDTA3Na; pentetic acid salts (diethylenetriaminepentaacetate salts); phytic acid; phosphonic acids such as etidronic acid, and salts thereof such as sodium salts; sodium oxalate; polyamino acids such as polyaspartic acid and polyglutamic acid; poly(sodium phosphate), sodium metaphosphate, phosphoric acid; sodium citrate, citric acid, alanine, dihydroxyethyl glycine, gluconic acid, ascorbic acid, succinic acid and tartaric acid.

Preferred examples of the pH adjusting agents, the acids and the alkalis include citric acid, sodium citrate, lactic acid, sodium lactate, potassium lactate, glycolic acid, succinic acid, acetic acid, sodium acetate, malic acid, tartaric acid, fumaric acid, phosphoric acid, hydrochloric acid, sulfuric acid, monoethanolamine, diethanolamine, triethanolamine, isopropanolamine, triisopropanolamine, 2-amino-2-methyl-1,3-propane diol, 2-amino-2-hydroxymethyl-1,3-propane diol, arginine, sodium hydroxide, potassium hydroxide, aqueous ammonia, guanidine carbonate and ammonium carbonate.

Preferred examples of the powders include inorganic powders, for example, inorganic powders of various sizes and shapes such as mica, talc, kaolin, sericite, montmorillonite, kaolinite, mica, muscovite, phlogopite, synthetic mica, lepidolite, biotite, vermiculite, magnesium carbonate, calcium carbonate, aluminum silicate, barium silicate, calcium silicate, magnesium silicate, strontium silicate, metal tungstate salts, magnesium, zeolite, barium sulfate, calcined calcium sulfate, calcium phosphate, fluoroapatite, hydroxyapatite, ceramic powder, bentonite, smectite, clay, mud, metallic soaps (for example, zinc myristate, calcium palmitate, aluminum stearate), calcium carbonate, red iron oxide, yellow iron oxide, black iron oxide, ultramarine blue, Prussian blue, carbon black, titanium oxide, fine particles and ultrafine particles of titanium oxide, zinc oxide, fine particles and ultrafine particles of zinc oxide, alumina, silica, fumed silica (ultrafine silicic anhydride particles), titanated mica, fish scale flakes, boron nitride, photochromic pigments, synthetic fluorine phlogopite, fine particle complex powder, gold and aluminum, and powders obtained by hydrophobizing or hydrophilizing the above powders by treatment with various kinds of surface treatment agents such as silicones including hydrogen silicone and cyclic hydrogen silicone, and other silane or titanium coupling agents; organic powders of various sizes and shapes such as starch, cellulose, Nylon powder, polyethylene powder, polymethyl methacrylate powder, polystyrene powder, styrene-acrylic acid copolymer resin powder, polyester powder, benzoguanamine resin powder, polyethylene terephthalate-polymethyl methacrylate laminated powder, polyethylene terephthalate-aluminum-epoxy laminated powder, etc., urethane powder, silicone powder and Teflon (registered trademark) powder, powders obtained by surface treatment of the above powders, and organic inorganic composite powders.

Preferred examples of the inorganic salts include sodium chloride-containing salts such as common salt, normal salt, rock salt, sea salt and natural salt; potassium chloride, aluminum chloride, calcium chloride, magnesium chloride, bittern, zinc chloride, ammonium chloride; sodium sulfate, aluminum sulfate, aluminum sulfate-potassium (alum), aluminum sulfate-ammonium, barium sulfate, calcium sulfate, potassium sulfate, magnesium sulfate, zinc sulfate, iron sulfate, copper sulfate; sodium phosphates such as 1Na phosphate, 2Na phosphate and 3Na phosphate, potassium phosphates, calcium phosphates and magnesium phosphates.

Preferred examples of the ultraviolet absorbers include benzoic acid-based ultraviolet absorbers such as para-aminobenzoic acid, para-aminobenzoic acid monoglycerin ester, N,N-dipropoxy-para-aminobenzoic acid ethyl ester, N,N-diethoxy-para-aminobenzoic acid ethyl ester, N,N-dimethyl-para-aminobenzoic acid ethyl ester, N,N-dimethyl-para-aminobenzoic acid butyl ester and N,N-dimethyl-para-aminobenzoic acid ethyl ester; anthranilic acid-based ultraviolet absorbers such as homomenthyl-N-acetylanthranilate; salicylic acid-based ultraviolet absorbers such as salicylic acid and sodium salt thereof, amyl salicylate, menthyl salicylate, homomenthyl salicylate, octyl salicylate, phenyl salicylate, benzyl salicylate and p-isopropanolphenyl salicylate; cinnamic acid-based ultraviolet absorbers such as octyl cinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, 2-ethylhexyl p-methoxycinnamate (octyl para-methoxycinnamate), 2-ethoxyethyl-p-methoxycinnamate (cinoxate), cyclohexyl-p-methoxycinnamate, ethyl-$\alpha$-cyano-$\beta$-phenylcinnamate, 2-ethylhexyl $\alpha$-cyano-$\beta$-phenylcinnamate (octocrylene), glyceryl mono-2-ethylhexanoyl-diparamethoxycinnamate, and ferulic acid and derivatives thereof; benzophenone-based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzone-3), 2-hydroxy-4-methoxy-4'-methylbenzophenone, 2-hydroxy-4-methoxybenzophenone-5-sulfonic acid salt, 4-phenylbenzophenone, 2-ethylhexyl-4'-phenyl-benzophenone-2-carboxylate, 2-hydroxy-4-n-octoxybenzophenone and 4-hydroxy-3-carboxybenzophenone; 3-(4'-methylbenzylidene)-d,l-camphor, 3-benzylidene-d,l-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenyl)benzotriazole; dibenzalazine; dianisoylmethane; 5-(3,3-dimethyl-2-norbornylidene)-3-pentan-2-one; dibenzoylmethane derivatives such as 4-t-butylmethoxydibenzoylmethane; octyl triazone; urocanic acid and urocanic acid derivatives such as ethyl urocanate; 2-(2'-hydroxy-5'-methylphenyl)benzotriazole, 1-(3,4-dimethoxyphenyl)-4,4-dimethyl-1,3-pentanedione, hydantoin derivatives such as 2-ethylhexyl dimethoxybenzylidenedioxoimidazolidinepropionate, phenylbenzimidazole sulfonic acid, terephthalylidene dicamphor sulfonic acid, drometrizole trisiloxane, methyl anthranilate, rutin and derivatives thereof, and oryzanol and derivatives thereof.

Preferred examples of the whitening agents include hydroquinone glycosides and esters thereof such as arbutin and $\alpha$-arbutin; ascorbic acid derivatives such as ascorbic acid, ascorbic acid phosphoric acid ester salts including ascorbic acid phosphoric acid ester sodium salt and ascorbic acid phosphoric acid ester magnesium salt, ascorbic acid fatty acid esters including ascorbic acid tetraisopalmitic acid ester, ascorbic acid alkyl ethers including ascorbic acid ethyl ether, ascorbic acid glucosides and fatty acid esters thereof including ascorbic acid-2-glucoside, ascorbic acid sulfuric acid esters and tocopheryl ascorbyl phosphate; kojic acid, ellagic acid, tranexamic acid and derivatives thereof, ferulic acid and derivatives thereof, placenta extract, glutathione, oryzanol, butylresorcinol, and plant extracts such as oil-soluble chamomile extract, oil-soluble licorice extract, Chinese tamarisk extract and Saxifraga stolonifera extract.

Preferred examples of the vitamins and the derivatives thereof include vitamins A such as retinol, retinol acetate and retinol palmitate; vitamins B such as thiamine hydrochloride, thiamine sulfate, riboflavin, riboflavin acetate, pyridoxine hydrochloride, pyridoxine dioctanoate, pyridoxine dipalmitate, flavin adenine dinucleotide, cyanocobalamin, folic acids, nicotinic acids including nicotinamide-benzyl nicotinate, and cholines; vitamins C such as ascorbic acid and salts thereof such as sodium salt; vitamin D; vitamins E such as $\alpha$, $\beta$, $\gamma$ and $\delta$-tocopherols; other vitamins such as pantothenic acid and biotin; ascorbic acid derivatives such as ascorbic acid phosphoric acid ester salts including ascorbic acid phosphoric acid ester sodium salt and ascorbic acid phosphoric acid ester magnesium salt, ascorbic acid fatty acid esters including ascorbic acid tetraisopalmitic acid ester, ascorbyl stearate, ascorbyl palmitate and ascorbyl dipalmitate, ascorbic acid alkyl ethers including ascorbic acid ethyl ether, ascorbic acid glucosides and fatty acid esters thereof including ascorbic acid-2-glucoside, and ascorbyl tocopheryl phosphate; vitamin derivatives such as tocopherol derivatives including tocopherol nicotinate, tocopherol acetate, tocopherol linoleate, tocopherol ferulate and tocopherolphosphoric acid ester, tocotrienols, and other various kinds of vitamin derivatives.

Preferred examples of the hair-growth agents, the blood circulation promoters and the stimulants include plant extracts and tinctures such as swertia herb extract, capsicum tincture, ginger tincture, ginger extract and cantharis tincture; capsaicin, nonylic acid vanillylamide, zingerone, ichthammol, tannic acid, borneol, cyclandelate, cinnarizine, tolazoline, acetylcholine, verapamil, cepharanthine, γ-oryzanol, vitamin E and derivatives such as nicotinic acid tocopherol and tocopherol acetate, γ-oryzanol, nicotinic acid and derivatives such as nicotinic acid amide, nicotinic acid benzyl ester, inositol hexanicotinate and nicotinyl alcohol, allantoin, photosensitizer 301, photosensitizer 401, carpronium chloride, pentadecanoic acid monoglyceride, flavanonol derivatives, stigmasterol or stigmastanol and glycosides thereof, and minoxidil.

Preferred examples of the hormones include estradiol, estrone, ethinyl estradiol, cortisone, hydrocortisone and prednisone. Preferred examples of other medicinal agents such as anti-wrinkle agents, anti-aging agents, tightening agents, cooling sensation agents, warming sensation agents, wound healing accelerators, irritation mitigating agents, analgesics and cell activators include retinols, retinoic acids, tocopheryl retinoate; lactic acid, glycolic acid, gluconic acid, fruit acid and salicylic acid, and derivatives thereof such as glycosides and esterified products, α- or β-hydroxy acids and derivatives thereof such as hydroxycapric acid, long-chain α-hydroxy fatty acid and long-chain α-hydroxy fatty acid cholesteryl; γ-aminobutyric acid, γ-amino-β-hydroxybutyric acid; carnitine; carnosine; creatine; ceramides, sphingosines; caffeine and xanthine, and derivatives thereof; antioxidants and active oxygen scavengers such as coenzyme Q10, carotene, lycopene, astaxanthin, lutein, α-lipoic acid, platinum nanocolloids and fullerenes; catechins; flavones such as quercetin; isoflavones; gallic acid and ester sugar derivatives; polyphenols such as tannin, sesamin, protoanthocyanidin, chlorogenic acid and apple polyphenol; rutin and derivatives such as glycosides; hesperidin and derivatives such as glycosides; lignan glycosides; licorice extract-related substances such as glabridin, glabrene, liquiritin and isoliquiritin; lactoferrin; shogaol, gingerol; fragrance substances and derivatives thereof such as menthol and cedrol; capsaicin and vanillin, and derivatives thereof; insect repellents such as diethyltoluamide; and complexes of physiologically active substances and cyclodextrins.

Preferred examples of the plant, animal and microbial extracts include such extracts as iris extract, Angelica keiskei extract, hiba arborvitae extract, asparagus extract, avocado extract, sweet hydrangea leaf extract, almond extract, althea extract, arnica extract, aloe extract, apricot extract, apricot kernel extract, ginkgo biloba extract, Artemisia Capillaris Flower extract, fennel extract, turmeric extract, oolong tea extract, bearberry leaf extract, rose fruit extract, Echinacea angustifolia leaf extract, isodon japonica extract, scutellariae radix extract, cork tree bark extract, coptis rhizome extract, barley extract, Panax ginseng extract, Hypericum erectum extract, Lamium album extract, ononis spinosa extract, Nasturtium officinale extract, orange extract, seawater dried product, seaweed extract, persimmon leaf extract, Pyracantha Fortuneana Fruit extract, hydrolyzed elastin, hydrolyzed wheat powder, hydrolyzed silk, pueraria root extract, chamomilla extract, oil-soluble chamomilla extract, carrot extract, Artemisia capillaris extract, wild oats extract, Hibiscus sabdariffa extract, licorice extract, oil-soluble licorice extract, kiwi fruit extract, kiou extract, Jew's Ear Fungus extract, cinchona extract, cucumber extract, paulownia leaf extract, guanosine, guava extract, Sophora root extract, gardenia extract, Sasa albo-marginata extract, Sophora angustifolia extract, walnut extract, chestnut extract, grapefruit extract, clematis extract, black rice extract, brown sugar extract, black vinegar, chlorella extract, mulberry extract, Gentiana lutea extract, Geranium thunbergii extract, black tea extract, yeast extract, magnolia bark extract, coffee extract, burdock extract, rice extract, rice fermented extract, rice bran fermented extract, rice germ oil, comfrey extract, collagen, cowberry extract, Asiasari radix extract, bupleuri radix extract, umbilical cord extract, saffron extract, salvia extract, common soapwort extract, sasa extract, hawthorn extract, silkworm droppings extract, Zanthoxylum piperitum extract, shiitake mushroom extract, Rehmannia glutinosa extract, Lithospermum root extract, perilla extract, Tilia japonica extract, Filipendula multijuga extract, Jatoba extract, peony extract, ginger extract, Calamus root extract, white birch extract, snow fungus extract, common horsetail extract, stevia extract, stevia fermented product, Chinese tamarisk extract, English ivy extract, Crataegus oxyacantha extract, Sambucus nigra extract, Achillea Millefolium extract, Mentha piperita extract, sage extract, common mallow extract, cnidium rhizome extract, swertia herb extract, mulberry bark extract, rhubarb extract, soybean extract, jujuba extract, thyme extract, dandelion extract, lichen extract, tea extract, clove extract, cogongrass extract, citrus unshiu peel extract, tea tree oil, sweet tea extract, capsicum extract, Angelica acutiloba extract, Calendula officinalis extract, peach kernel extract, spruce extract, chameleon plant extract, tomato extract, fermented soybeans extract, carrot extract, garlic extract, bramble extract, hibiscus extract, ophiopogon tuber extract, lotus extract, parsley extract, birch extract, honey, hamamelis extract, parietaria extract, Isodon japonicus extract, bisabolol, Japanese cypress extract, Lactobacillus bifidus extract, loquat extract, coltsfoot extract, Petasites japonicus extract, Poria Sclerotium extract, butcher's broom extract, grape extract, grape seed extract, propolis, loofah extract, safflower extract, peppermint extract, Tilia miqueliana extract, Paeonia suffruticosa extract, hop extract, rugosa rose extract, pine tree extract, horse chestnut extract, skunk cabbage extract, washnut extract, lemon balm extract, Cladosiphon okamuranus extract, peach extract, cornflower extract, eucalyptus extract, saxifraga extract, Citrus junos extract, lily extract, coix seed extract, mugwort extract, lavender extract, green tea extract, eggshell membrane extract, apple extract, rooibos tea extract, litchi extract, lettuce extract, lemon extract, forsythia extract, Astragalus sinicus extract, rose extract, rosemary extract, Anthemis nobilis extract, royal jelly extract and burnet extract.

Examples of the antipruritic agents include diphenhydramine hydrochloride, chlorpheniramine maleate, camphor and substance-P inhibitors.

Examples of the keratin exfoliating/dissolving agents include salicylic acid, sulfur, resorcin, selenium sulfide and pyridoxine.

Examples of the antiperspirants include chlorohydroxyaluminum, aluminum chloride, zinc oxide and zinc paraphenolsulfonate.

Examples of the algefacients include menthol and methyl salicylate.

Examples of the astringents include citric acid, tartaric acid, lactic acid, aluminum-potassium sulfate and tannic acid.

Examples of the enzymes include superoxide dismutase, catalase, lysozyme chloride, lipase, papain, pancreatin and protease.

Preferred examples of the nucleic acids include ribonucleic acids and salts thereof, deoxyribonucleic acids and salts thereof, and adenosine triphosphate disodium.

Preferred examples of the fragrances include synthetic fragrances and natural fragrances, and various kinds of mixed fragrances such as acetyl cedrene, amylcinnamaldehyde, allyl amyl glycolate, β-ionone, Iso E Super, isobutylquinoline, iris oil, irone, indole, ylang ylang oil, undecanal, undecenal, γ-undecalactone, estragole, eugenol, oakmoss, opoponax resinoid, orange oil, eugenol, auranthiol, Galaxolide, carvacrol, L-carvone, camphor, canon, carrot seed oil, clove oil, methyl cinnamate, geraniol, geranyl nitrile, isobornyl acetate, geranyl acetate, dimethyl benzyl carbinyl acetate, styrallyl acetate, cedryl acetate, terpinyl acetate, p-t-butylcyclohexyl acetate, vetiveryl acetate, benzyl acetate, linalyl acetate, isopentyl salicylate, benzyl salicylate, sandalwood oil, santalol, cyclamen aldehyde, cyclopentadecanolide, methyl dihydrojasmonate, dihydromyrcenol, jasmine absolute, jasmine lactone, cis-jasmone, citral, citronellol, citronellal, cinnamon bark oil, 1,8-cineole, cinnamaldehyde, styrax resinoid, cedarwood oil, cedrene, cedrol, celery seed oil, thyme oil, damascone, damascenone, thymol, tuberose absolute, decanal, decalactone, terpineol, γ-terpinene, Triplal, nerol, nonanal, 2,6-nonadienol, nonalactone, patchouli alcohol, vanilla absolute, vanillin, basil oil, patchouli oil, hydroxycitronellal, α-pinene, piperitone, phenethyl alcohol, phenylacetaldehyde, petitgrain oil, hexyl cinnamaldehyde, cis-3-hexenol, Peru balsam, vetiver oil, vetiverol, peppermint oil, pepper oil, heliotropin, bergamot oil, benzyl benzoate, borneol, myrrh resinoid, musk ketone, methyl nonyl acetaldehyde, γ-methyl ionone, menthol, L-menthol, L-menthone, eucalyptus oil, β-ionone, lime oil, lavender oil, D-limonene, linalool, Lyral, lilial, lemon oil, rose absolute, rose oxide, rose oil, rosemary oil and various essential oils.

Preferred examples of the colorants, the coloring agents, the dyes and the pigments include legal colorants such as Brown No. 201, Black No. 401, Purple No. 201, Purple No. 401, Blue No. 1, Blue No. 2, Blue No. 201, Blue No. 202, Blue No. 203, Blue No. 204, Blue No. 205, Blue No. 403, Blue No. 404, Green No. 201, Green No. 202, Green No. 204, Green No. 205, Green No. 3, Green No. 401, Green No. 402, Red No. 102, Red No. 104-1, Red No. 105-1, Red No. 106, Red No. 2, Red No. 201, Red No. 202, Red No. 203, Red No. 204, Red No. 205, Red No. 206, Red No. 207, Red No. 208, Red No. 213, Red No. 214, Red No. 215, Red No. 218, Red No. 219, Red No. 220, Red No. 221, Red No. 223, Red No. 225, Red No. 226, Red No. 227, Red No. 228, Red No. 230-1, Red No. 230-2, Red No. 231, Red No. 232, Red No. 3, Red No. 401, Red No. 404, Red No. 405, Red No. 501, Red No. 502, Red No. 503, Red No. 504, Red No. 505, Red No. 506, Orange No. 201, Orange No. 203, Orange No. 204, Orange No. 205, Orange No. 206, Orange No. 207, Orange No. 401, Orange No. 402, Orange No. 403, Yellow No. 201, Yellow No. 202-1, Yellow No. 202-2, Yellow No. 203, Yellow No. 204, Yellow No. 205, Yellow No. 4, Yellow No. 401, Yellow No. 402, Yellow No. 403-1, Yellow No. 404, Yellow No. 405, Yellow No. 406, Yellow No. 407 and Yellow No. 5; other acid dyes such as Acid Red 14; basic dyes such as Arianor Sienna Brown, Arianor Madder Red, Arianor Steel Blue and Arianor Straw Yellow; nitro dyes such as HC Yellow 2, HC Yellow 5, HC Red 3, 4-hydroxypropylamino-3-nitrophenol, N,N'-bis(2-hydroxyethyl)-2-nitro-p-phenylenediamine, HC Blue 2 and Basic Blue 26; disperse dyes; inorganic white pigments such as titanium dioxide and zinc oxide; inorganic red pigments such as iron oxide (rouge) and iron titanate; inorganic brown pigments such as γ-iron oxide; inorganic yellow pigments such as yellow iron oxide and ocher; inorganic black pigments such as black iron oxide and low-order titanium oxide; inorganic purple pigments such as mango violet and cobalt violet; inorganic green pigments such as chromium oxide, chromium hydroxide and cobalt titanate; inorganic blue pigments such as ultramarine blue and Prussian blue; pearl pigments such as titanium oxide coated mica, titanium oxide coated bismuth oxychloride, titanium oxide coated talc, colored titanium oxide coated mica, bismuth oxychloride and fish scale flakes; metal powder pigments such as aluminum powder, copper powder and gold; surface-treated inorganic and metal powder pigments; organic pigments such as zirconium, barium and aluminum lakes; surface-treated organic pigments; natural colorants and dyes such as astaxanthin, anthraquinones including alizarin, anthocyanidin, β-carotene, carotenal, capsanthin, chalcone, carthamine, quercetin, crocin, chlorophyll, curcumin, cochineal, naphthoquinones including shikonin, bixin, flavones, betacyanidin, henna, hemoglobin, lycopene, riboflavin and rutin; oxidative dye intermediates and couplers such as p-phenylenediamine, toluene-2,5-diamine, o-, m- or p-aminophenol, m-phenylenediamine, 5-amino-2-methylphenol, resorcin, 1-naphthol and 2,6-diaminopyridine, and salts thereof; autoxidative dyes such as indoline; and dihydroxyacetone.

Preferred examples of the antiphlogistics and the anti-inflammatory agents include glycyrrhizic acid and derivatives thereof, glycyrrhetinic acid derivatives, salicylic acid derivatives, hinokitiol, guaiazulene, allantoin, indomethacin, ketoprofen, ibuprofen, diclofenac, loxoprofen, celecoxib, infliximab, etanercept, zinc oxide, hydrocortisone acetate, prednisone, diphenhydramine hydrochloride, chlorpheniramine maleate; and plant extracts such as peach leaf extract and mugwort leaf extract.

Preferred examples of the anti-asthmatic agents, the anti-chronic obstructive pulmonary disease agents, the anti-allergic agents and the immunomodulators include aminophylline, theophyllines, steroids (fluticasone, beclomethasone, etc.), leukotriene antagonists, thromboxane inhibitors, Intal, $\beta2$ stimulants (formoterol, salmeterol, albuterol, tulobuterol, clenbuterol, epinephrine, etc.), tiotropium, ipratropium, dextromethorphan, dimemorphan, bromhexine, Tranilast, ketotifen, azelastine, cetirizine, chlorpheniramine, mequitazine, tacrolimus, cyclosporin, sirolimus, methotrexate, cytokine regulators, interferon, omalizumab and protein/antibody preparations.

Preferred examples of the anti-infective agents and the antifungal agents include oseltamivir, zanamivir and itraconazole. In addition to the additives described above, other known ingredients may be added in known combinations and in known mixing ratios and amounts, with examples including cosmetic ingredients, pharmaceutical ingredients and food ingredients described in literature such as The Japanese Standards of Cosmetic Ingredients, The Japanese Cosmetic Ingredients Codex by Category, the list of ingredient labeling names of Japan Cosmetic Industry Association, The INCI Dictionary (The International Cosmetic Ingredient Dictionary and Handbook), The Japanese Standards of Quasidrug Ingredients, The Japanese Pharmacopoeia, The Japanese Pharmaceutical Excipients, and The Japan's Specifications and Standards for Food Additives, and ingredients described in patent gazettes and patent gazettes of unexamined patent applications (including publication gazettes and republications) of Japan and foreign countries whose International Patent Classification IPC belong to the classifications of A61K7 and A61K8.


Examples

[Reference Synthesis Example 1: 10 mass% Microcrystalline cellulose dispersion]

8 kg of microcrystalline cellulose (Comprecel M101 manufactured by FUSHIMI Pharmaceutical Co., Ltd.) was dispersed in 72 kg of ion-exchanged water and was pulverized one time with wet pulverization device Star Burst (manufactured by SUGINO MACHINE LIMITED) at 150 MPa. The title 10 mass% microcrystalline cellulose dispersion was thus obtained.

[Reference Synthesis Example 2: 4 mass% Microcrystalline cellulose dispersion]

0.6 kg of microcrystalline cellulose (Comprecel M101 manufactured by FUSHIMI Pharmaceutical Co., Ltd.) was dispersed in 14.4 kg of ion-exchanged water and was pulverized ten times with wet pulverization device Star Burst (manufactured by SUGINO MACHINE LIMITED) at 150 MPa. The title 4 mass% microcrystalline cellulose dispersion was thus obtained.

[Example 1: Particles having cellulose:yellow iron oxide = 1:1 w/w]

A dispersion was obtained by mixing 450 g of the microcrystalline cellulose dispersion obtained in Reference Synthesis Example 1, 45 g of yellow iron oxide (LL-100HP manufactured by Titan Kogyo, Ltd.) and 5 g of pure water. The dispersion obtained was added to MDL-050B (manufactured by Fujisaki Electric Co., Ltd.) equipped with SE nozzle and was sprayed at a hot air inlet temperature of 160°C, an air supply volume of 1.00 m$^3$/min, a nozzle air flow rate of 40 NL/min and a liquid volume of 30 mL/min. 45 g of powder as the title particles was thus obtained.

[Example 2: Particles having cellulose:yellow iron oxide = 1:2 w/w]

A dispersion was obtained by mixing 300 g of the microcrystalline cellulose dispersion obtained in Reference Synthesis Example 1, 60 g of yellow iron oxide (LL-100HP manufactured by Titan Kogyo, Ltd.) and 140 g of pure water. The dispersion obtained was added to MDL-050B (manufactured by Fujisaki Electric Co., Ltd.) equipped with SE nozzle and was sprayed at a hot air inlet temperature of 160°C, an air supply volume of 1.00 m$^3$/min, a nozzle air flow rate of 40 NL/min and a liquid volume of 30 mL/min. 52 g of powder as the title particles was thus obtained.

[Example 3: Particles having cellulose:yellow iron oxide = 1:4 w/w]

A dispersion was obtained by mixing 360 g of the microcrystalline cellulose dispersion obtained in Reference Synthesis Example 1, 144 g of yellow iron oxide (LL-100HP manufactured by Titan Kogyo, Ltd.) and 496 g of pure water. The dispersion obtained was added to MDL-050B (manufactured by Fujisaki Electric Co., Ltd.) equipped with SE nozzle and was sprayed at a hot air inlet temperature of 160°C, an air supply volume of 1.00 m³/min, a nozzle air flow rate of 40 NL/min and a liquid volume of 30 mL/min. 149 g of powder as the title particles was thus obtained.

[Example 4: Particles having cellulose:yellow iron oxide = 1:4 w/w]

A dispersion was obtained by mixing 108 g of the microcrystalline cellulose dispersion obtained in Reference Synthesis Example 2, 17.3 g of yellow iron oxide (LL-100HP manufactured by Titan Kogyo, Ltd.) and 54.7 g of pure water. The dispersion obtained was added to MDL-050B (manufactured by Fujisaki Electric Co., Ltd.) equipped with SE nozzle and was sprayed at a hot air inlet temperature of 160°C, an air supply volume of 1.00 m³/min, a nozzle air flow rate of 40 NL/min and a liquid volume of 30 mL/min. 3 g of powder as the title particles was thus obtained.

[Example 5: Particles having cellulose:red iron oxide = 1:2 w/w]

A dispersion was obtained by mixing 600 g of the microcrystalline cellulose dispersion obtained in Reference Synthesis Example 1, 120 g of red iron oxide (R-516HP manufactured by Titan Kogyo, Ltd.) and 280 g of pure water. The dispersion obtained was added to MDL-050B (manufactured by Fujisaki Electric Co., Ltd.) equipped with SE nozzle and was sprayed at a hot air inlet temperature of 160°C, an air supply volume of 1.00 m³/min, a nozzle air flow rate of 40 NL/min and a liquid volume of 30 mL/min. 77 g of powder as the title particles was thus obtained.

[Example 6: Particles having cellulose:red iron oxide = 1:4 w/w]

A dispersion was obtained by mixing 360 g of the microcrystalline cellulose dispersion obtained in Reference Synthesis Example 1, 144 g of red iron oxide (R-516HP manufactured by Titan Kogyo, Ltd.) and 496 g of pure water. The dispersion obtained was added to MDL-050B (manufactured by Fujisaki Electric Co., Ltd.) equipped with SE nozzle and was sprayed at a hot air inlet temperature of 160°C, an air supply volume of 1.00 m³/min, a nozzle air flow rate of 40 NL/min and a liquid volume of 30 mL/min. 109 g of powder as the title particles was thus obtained.

[Example 7: Particles having cellulose:red iron oxide = 1:9 w/w]

A dispersion was obtained by mixing 180 g of the microcrystalline cellulose dispersion obtained in Reference Synthesis Example 1, 162 g of red iron oxide (R-516HP manufactured by Titan Kogyo, Ltd.) and 658 g of pure water. The dispersion obtained was added to MDL-050B (manufactured by Fujisaki Electric Co., Ltd.) equipped with SE nozzle and was sprayed at a hot air inlet temperature of 160°C, an air supply volume of 1.00 m³/min, a nozzle air flow rate of 40 NL/min and a liquid volume of 30 mL/min. 112 g of powder as the title particles was thus obtained.

[Reference Synthesis Example 3: 5 mass% Dispersion having microcrystalline cellulose:boron nitride = 1:4 w/w]

0.25 kg of microcrystalline cellulose (Comprecel M101 manufactured by Fushimi Pharmaceutical Co., Ltd.) and 1.00 kg of boron nitride (RonaFlair (registered trademark) Boroneige SF-3 manufactured by Merck Performance Materials Co., Ltd.) were dispersed in 23.75 kg of ion-exchanged water and were pulverized three times with wet pulverization device Star Burst (manufactured by SUGINO MACHINE LIMITED) at 150 MPa. The title dispersion was thus obtained.

[Example 8: Particles having cellulose:boron nitride = 1:4 w/w]

The dispersion obtained in Reference Synthesis Example 3 was added to spray dryer CL-8 (manufactured by OHKAWARA KAKOHKI CO., LTD.) equipped with RJ-5 nozzle (manufactured by OHKAWARA KAKOHKI CO., LTD.) and was spray-dried at a feedstock throughput of 2.0 kg/h, a spray pressure of 0.1 MPa, a hot air inlet temperature of 170°C, an outlet temperature of 82°C and a cyclone differential pressure of 0.5 kPa for 2 hours and 47 minutes. 139 g of powder as the title particles was thus obtained.

[Reference Synthesis Example 4: 5 mass% Microcrystalline cellulose dispersion]

5 kg of microcrystalline cellulose (Comprecel M101 manufactured by FUSHIMI Pharmaceutical Co., Ltd.) was dispersed in 95 kg of ion-exchanged water and was pulverized five times with wet pulverization device Star Burst (manufactured by SUGINO MACHINE LIMITED) at 150 MPa. The title 5 mass% microcrystalline cellulose dispersion was thus obtained.

[Reference Synthesis Example 5: 5 mass% Microcrystalline cellulose dispersion]

2.5 kg of microcrystalline cellulose (Comprecel M101 manufactured by FUSHIMI Pharmaceutical Co., Ltd.) was dispersed in 47.5 kg of ion-exchanged water and was pulverized five times with wet pulverization device Star Burst (manufactured by SUGINO MACHINE LIMITED) at 150 MPa. The title 5 mass% microcrystalline cellulose dispersion was thus obtained.

[Reference Synthesis Example 6: 5 mass% Microcrystalline cellulose dispersion]

4.0 kg of microcrystalline cellulose (Comprecel M101 manufactured by FUSHIMI Pharmaceutical Co., Ltd.) was dispersed in 76.0 kg of ion-exchanged water and was pulverized five times with wet pulverization device Star Burst (manufactured by SUGINO MACHINE LIMITED) at 150 MPa. The title 5 mass% microcrystalline cellulose dispersion was thus obtained.

[Example 9: Particles having cellulose:titanium oxide = 1:4 w/w]

A dispersion was obtained by mixing 12 kg of the microcrystalline cellulose dispersion obtained in Reference Synthesis Example 4, 2.4 kg of titanium oxide (MKR-1 manufactured by SAKAI CHEMICAL INDUSTRY CO., LTD.) and 5.6 kg of ion-exchanged water. The dispersion obtained was added to spray dryer RL-5 (manufactured by OHKAWARA KAKOHKI CO., LTD.) equipped with RJ-10 nozzle (manufactured by OHKAWARA KAKOHKI CO., LTD.), and 15.29 kg of the dispersion was spray-dried at a feedstock throughput of 9.6 kg/h, a spray pressure of 0.3 MPa, a hot air inlet temperature of 250°C, an outlet temperature of 96°C and a cyclone differential pressure of 1.7 kPa. 1.0 kg of powder as the title particles was thus obtained.

[Example 10: Particles having cellulose:yellow iron oxide = 1:4 w/w]

A dispersion was obtained by mixing 12 kg of the microcrystalline cellulose dispersion obtained in Reference Synthesis Example 5, 2.4 kg of yellow iron oxide (LL-100HP manufactured by Titan Kogyo, Ltd.) and 10.6 kg of ion-exchanged water. The dispersion obtained was added to spray dryer RL-5 (manufactured by OHKAWARA KAKOHKI CO., LTD.) equipped with RJ-10 nozzle (manufactured by OHKAWARA KAKOHKI CO., LTD.), and 5.35 kg of the dispersion was spray-dried at a feedstock throughput of 8.7 kg/h, a spray pressure of 0.3 MPa, a hot air inlet temperature of 250°C, an outlet temperature of 96°C and a cyclone differential pressure of 1.7 kPa. 0.49 kg of powder as the title particles was thus obtained.

[Example 11: Particles having cellulose:yellow iron oxide = 1:4 w/w]

A dispersion was obtained by mixing 12 kg of the microcrystalline cellulose dispersion obtained in Reference Synthesis Example 5, 2.4 kg of yellow iron oxide (LL-100HP manufactured by Titan Kogyo, Ltd.) and 10.6 kg of ion-exchanged water. The dispersion obtained was added to spray dryer RL-5 (manufactured by OHKAWARA KAKOHKI CO., LTD.) equipped with RJ-10 nozzle (manufactured by OHKAWARA KAKOHKI CO., LTD.), and 11.39 kg of the dispersion was spray-dried at a feedstock throughput of 8.7 kg/h, a spray pressure of 0.2 MPa, a hot air inlet temperature of 250°C, an outlet temperature of 96°C and a cyclone differential pressure of 1.7 kPa. 1.17 kg of powder as the title particles was thus obtained.

[Example 12: Particles having cellulose:yellow iron oxide = 1:4 w/w]

A dispersion was obtained by mixing 10.4 kg of the microcrystalline cellulose dispersion obtained in Reference Synthesis Example 6, 2.08 kg of yellow iron oxide (LL-100HP manufactured by Titan Kogyo, Ltd.) and 7.52 kg of ion-exchanged water. The dispersion obtained was added to spray dryer RL-5 (manufactured by OHKAWARA KAKOHKI CO., LTD.) equipped with RJ-10 nozzle (manufactured by OHKAWARA KAKOHKI CO., LTD.), and 19.72 kg of the dispersion was spray-dried at a feedstock throughput of 9.6 kg/h, a spray pressure of 0.2 MPa, a hot air inlet temperature of 250°C, an outlet temperature of 102°C and a cyclone differential pressure of 1.7 kPa. 2.15 kg of powder as the title particles was thus obtained.

[Example 13: Particles having cellulose:red iron oxide = 1:4 w/w]

A dispersion was obtained by mixing 13.6 kg of the microcrystalline cellulose dispersion obtained in Reference Synthesis Example 6, 2.72 kg of red iron oxide (C33-8001 manufactured by DIC CORPORATION) and 5.94 kg of ion-exchanged water. The dispersion obtained was added to spray dryer RL-5 (manufactured by OHKAWARA KAKOHKI CO., LTD.) equipped with RJ-10 nozzle (manufactured by OHKAWARA KAKOHKI CO., LTD.), and 12.64 kg of the dispersion was spray-dried at a feedstock throughput of 9.5 kg/h, a spray pressure of 0.1 MPa, a hot air inlet temperature of 250°C, an outlet temperature of 101°C and a cyclone differential pressure of 1.7 kPa. 1.5 kg of powder as the title particles was thus obtained.

[Example 14: Particles having cellulose:red iron oxide = 1:4 w/w]

A dispersion was obtained by mixing 12 kg of the microcrystalline cellulose dispersion obtained in Reference Synthesis Example 6, 2.4 kg of red iron oxide (R-516HP manufactured by Titan Kogyo, Ltd.) and 10.6 kg of ion-exchanged water. The dispersion obtained was added to spray dryer RL-5 (manufactured by OHKAWARA KAKOHKI CO., LTD.) equipped with RJ-10 nozzle (manufactured by OHKAWARA KAKOHKI CO., LTD.), and 8.74 kg of the dispersion was spray-dried at a feedstock throughput of 9.5 kg/h, a spray pressure of 0.2 MPa, a hot air inlet temperature of 250°C, an outlet temperature of 99°C and a cyclone differential pressure of 1.7 kPa. 0.91 kg of powder as the title particles was thus obtained.

[Example 15: Particles having cellulose:black iron oxide = 1:4 w/w]

A dispersion was obtained by mixing 13.6 kg of the microcrystalline cellulose dispersion obtained in Reference Synthesis Example 6, 2.72 kg of black iron oxide (BL-100HP manufactured by Titan Kogyo, Ltd.) and 3.68 kg of ion-exchanged water. The dispersion obtained was added to spray dryer RL-5 (manufactured by OHKAWARA KAKOHKI CO., LTD.) equipped with RJ-10 nozzle (manufactured by OHKAWARA KAKOHKI CO., LTD.), and 19.88 kg of the dispersion was spray-dried at a feedstock throughput of 9.7 kg/h, a spray pressure of 0.2 MPa, a hot air inlet temperature of 250°C, an outlet temperature of 97°C and a cyclone differential pressure of 1.7 kPa. 3.02 kg of powder as the title particles was thus obtained.

[Reference Synthesis Example 7: 20 mass% Microcrystalline cellulose/barium sulfate dispersion]

0.6 kg of microcrystalline cellulose (Comprecel M101 manufactured by Fushimi Pharmaceutical Co., Ltd.) and 2.4 kg of barium sulfate (platy barium sulfate H, manufactured by SAKAI CHEMICAL INDUSTRY CO., LTD.) were dispersed in 12 kg of ion-exchanged water and were pulverized four times with wet pulverization device Star Burst (manufactured by SUGINO MACHINE LIMITED) at 150 MPa. The title 20 mass% microcrystalline cellulose/barium sulfate dispersion was thus obtained.

[Example 16: Particles having cellulose:barium sulfate = 1:4 w/w]

The microcrystalline cellulose/barium sulfate dispersion obtained in Reference Synthesis Example 7 was added to spray dryer CL-8 (manufactured by OHKAWARA KAKOHKI CO., LTD.) equipped with RJ-5 nozzle (manufactured by OHKAWARA KAKOHKI CO., LTD.) and was spray-dried at a feedstock throughput of 2.0 kg/h, a spray pressure of 0.2 MPa, a hot air inlet temperature of 170°C, an outlet temperature of 93°C and a cyclone differential pressure of 0.5 kPa for 13 minutes. 60 g of powder as the title particles was thus obtained.

[Evaluation Example 1: Observation of particle morphology]

The particles obtained in Examples 1 to 7 and 9 to 15 were each attached to carbon tape, and the morphology was observed with scanning electron microscope Miniscope (registered trademark) TM3000 (manufactured by Hitachi High-Tech Corporation). The results of particle morphology observation are illustrated in Figs. 1(a) to 1(n), respectively. Further, the particles obtained in Examples 8, 9 and 12 to 16 were each attached to carbon tape, and the morphology was observed with field emission scanning electron microscope JSM-7400F (manufactured by JEOL Ltd.) at an acceleration voltage of 0.7 kV or 1.0 kV and an amount of current of 10 $\mu$V. The results of particle morphology observation are illustrated in Figs. 1(o) to 1(u), respectively.

[Evaluation Example 2: Evaluation of particle size]

With use of scattering-type particle size distribution analyzer LA-960 (manufactured by HORIBA, Ltd.), the concentration of a sample solution of the particles obtained in any of Examples 1 to 16 was adjusted while circulating the sample solution at a circulation speed of "3" and a stirring rate of "2" so that the transmittance of a semiconductor laser beam (650 nm) and that of an LED light (405 nm) would be 90.0% or less. The sample solution thus obtained was ultrasonicated at an ultrasonic wave intensity of "3" for 1 minute, and thereafter the particle size was measured. The results are sequentially described in Figs. 2(a) to 2(p).

[Evaluation Example 3: Method for preparing cross section of particle and method for calculating void ratio]

Silver paste was applied onto a silicon wafer, and the particles obtained in any of Examples 3 to 7, 9 and 12 to 15 were sprinkled. The excess particles were removed by air blowing, and platinum was deposited using ion sputter MC1000 (manufactured by Hitachi, Ltd.) at 15 mA for 100 seconds. A sample was thus fabricated. A cross section of the sample was prepared and observed with focused ion beam-scanning electron microscope (FIB-SEM) Helios NanoLab G3 (manufactured by Thermo Fisher Scientific) at an acceleration voltage of 1 kV and a current value of 0.1 nA. The reflected electron image obtained by the observation was analyzed with Avizo 9.5 software (manufactured by Thermo Fisher Scientific), and the void ratio was calculated using the equation below. The test was performed three times.

$$\text{Void Ratio}(\%) = \frac{\text{Void area}}{\text{Sectional area}} \times 100\%$$

In the equation, the void area indicates the total area of voids in the sectional image of the particle, and the sectional area indicates the area of the entire cross section in the sectional image of the particle.

The sectional observation images of the particles obtained in Examples 3 to 7, 9 and 12 to 15 are illustrated in Figs. 3(a) to 12(a). The images highlighting the entire cross section of the particle used in the calculation of void ratio, and the areas of the cross section (the sectional areas) are shown in Figs. 3(b) to 12(b). The images highlighting the voids in the cross section of the particle, and the areas of the voids (the void areas) are shown in Figs. 3(c) to 12(c). The average values of the void ratio of the particles are described in Table 1.

[Table 1]

| Evaluation sample | Void Ratio (%) | | | |
|---|---|---|---|---|
| | First | Second | Third | Average |
| Example 3 | 13.3 | 10.3 | 9.8 | 11.1 |
| Example 4 | 19.9 | 21.7 | 23. 2 | 21.6 |
| Example 5 | 24.9 | 21.3 | 24. 1 | 23.4 |
| Example 6 | 26.3 | 19.6 | 19.8 | 21.9 |
| Example 7 | 24.4 | 24.2 | 30.4 | 26. 3 |
| Example 9 | 15. 1 | 14.9 | 18.9 | 16.3 |
| Example 12 | 21.8 | 17.4 | 20. 2 | 19.8 |
| Example 13 | 14.4 | 14.2 | 22. 8 | 17. 1 |
| Example 14 | 23.0 | 23. 1 | 22. 5 | 22. 9 |
| Example 15 | 20.2 | 27.3 | 28. 5 | 25. 3 |

[Evaluation Example 4: Measurement of reflected light distribution]

Black drawing paper PI-N86D (manufactured by MARUAI Inc.) was attached onto microslide glass S111 (manufactured by Matsunami Glass Ind., Ltd.) using NICETACK (registered trademark) NW-10S (manufactured by NICHIBAN Co., Ltd.). Next, NICETACK (registered trademark) NW-10S was pasted onto the black drawing paper, and sample powder was pressed against the adhesive tape. The excess particles were removed with an air gun adjusted to a pressure of 0.2 MPa. The distribution of reflected light was measured with goniophotometer GP-5 (manufactured by MURAKAMI COLOR RESEARCH LABORATORY CO., LTD.). The measurement incident light was -45 degrees. The light scattering rate at an incident light of -45° was calculated in accordance with the following equation (1) (see WO 2010/092890).

$$\text{Light scattering rate }(\%) = \frac{(\text{Reflection intensity at }20° \text{ angle}/\cos 20°) + (\text{Reflection intensity at }70° \text{ angle}/\cos 70°)}{2\times (\text{Reflection intensity at }5° \text{ angle}/\cos 5°)} \times 100 \cdots (1)$$

The samples that were used were the particles obtained in Examples 1 to 7. Further used were yellow iron oxide (LL-100HP manufactured by Titan Kogyo, Ltd.) as Comparative Example 1; powder obtained by stirring and mixing microcrystalline cellulose powder (Comprecel M101 manufactured by Fushimi Pharmaceutical Co., Ltd.) and yellow iron oxide (LL-100HP manufactured by Titan Kogyo, Ltd.) in a mixing ratio of 1:4 (w/w) as Comparative Example 2; red iron oxide (R-516HP manufactured by Titan Kogyo, Ltd.) as Comparative Example 3; and powder obtained by stirring and mixing microcrystalline cellulose powder (Comprecel M101 manufactured by Fushimi Pharmaceutical Co., Ltd.) and red iron oxide (R-516HP manufactured by Titan Kogyo, Ltd.) in a mixing ratio of 1:4 (w/w) as Comparative Example 4. The light scattering rates of the samples at an incident light of -45 degrees are described in Table 2.

[Table 2]

| Results at incident light of -45° | | | | |
|---|---|---|---|---|
| Evaluation sample | Reflection intensity at 5° angle | Reflection intensity at 20° angle | Reflection intensity at 70° angle | Light scattering rate (%) |
| Example 1 | 78. 35 | 74.92 | 38.11 | 121.52 |
| Example 2 | 81. 85 | 77.69 | 38.33 | 118.51 |
| Example 3 | 50.97 | 48.95 | 26.50 | 126.62 |
| Example 4 | 80.46 | 76.35 | 38.46 | 119.91 |
| Example 5 | 49.74 | 46. 79 | 26. 15 | 126.43 |
| Example 6 | 49. 70 | 46.27 | 23.22 | 117.39 |
| Example 7 | 50.09 | 47.79 | 25.93 | 125.96 |
| Comparative Example 1 | 62.94 | 64. 14 | 38.84 | 143.89 |
| Comparative Example 2 | 72.93 | 70. 6 | 39.32 | 129.83 |
| Comparative Example 3 | 64.02 | 63.02 | 39.68 | 142.44 |
| Comparative Example 4 | 72.67 | 68.96 | 38.99 | 128.44 |

[Evaluation Example 5: Evaluation of tint of particles]

An aluminum ring (28 φ) for powder sample (manufactured by Rigaku Corporation) was set on weighing paper, and 1.1 g of evaluation particles were weighed inside the ring. Next, the sample that had been weighed in the aluminum ring was compressed using desktop test press SA-302-I-S (manufactured by TESTER SANGYO CO,. LTD.) at a pressure of 30 kgf/cm$^2$ for 1 minute, thus forming a pellet-shaped measurement sample. The surface of the sample was analyzed with portable colorimeter WR10 (manufactured by Shenzhen Wave Optoelectronics Technology Co., Ltd.). The evaluation particles that were used were the particles obtained in Examples 3 and 6. Further, the powders used as Comparative Examples 1 to 4 in Evaluation Example 4 were used as Comparative Examples. The evaluation results of the samples are described in Table 3.

[Table 3]

| Evaluation sample | L* | a * | b* |
|---|---|---|---|
| Comparative Example 1 | 71.57 | 11. 92 | 52.69 |
| Comparative Example 2 | 68. 76 | 11. 94 | 51. 65 |
| Example 3 | 68.31 | 15. 11 | 54. 77 |
| Comparative Example 3 | 39.50 | 38. 53 | 42.83 |
| Comparative Example 4 | 37.25 | 37. 78 | 43. 78 |

(continued)

| Evaluation sample | L* | a * | b* |
|---|---|---|---|
| Example 6 | 37.89 | 38. 70 | 40.52 |

[Evaluation Example 6: Evaluation of tint and hiding power of particles]

Transparent optical adhesive sheet LUCIACS (registered trademark) CS9862UA (manufactured by NITTO DENKO CORPORATION) was attached onto a glass substrate (microslide glass S1111 manufactured by Matsunami Glass Ind., Ltd. or a 1 mm thick substrate manufactured by TOSHIN RIKO CO., LTD.). Next, evaluation particles were uniformly applied with a brush onto the transparent optical adhesive sheet LUCIACS (registered trademark) CS9862UA (manufactured by NITTO DENKO CORPORATION), and the excess particles were removed with a brush so that the predetermined amount of the particles would be attached (0.14 mg/cm$^2$ for Example 3, Comparative Example 1 and Comparative Example 2, 0.0875 mg/cm$^2$ for Example 6, Comparative Example 3 and Comparative Example 4, 0.2083 mg/cm$^2$ for Example 8, and 0.0694 mg/cm$^2$ for Comparative Example 5). The tint and the hiding power were measured by placing the slide glass coated with the sample onto white copy paper (A4 PPC PAPER High White) or black drawing paper PI-N86D (manufactured by MARUAI Inc.) and analyzing the sample with portable colorimeter WR10 (manufactured by Shenzhen Wave Optoelectronics Technology Co., Ltd.). The results of the measurement of brightness (L*), redness (a*) and yellowness (b*) of the samples are described in Table 4. Table 4 also describes the hiding power as the difference in L* value between on the white copy paper (A4 PPC PAPER High White) and on the black drawing paper PI-N86D (manufactured by MARUAI Inc.). The evaluation particles that were used were the particles obtained in Examples 3, 6 and 8. The powders used as Comparative Examples 1 to 4 in Evaluation Example 4 were also used as Comparative Examples. Further, boron nitride (RonaFlair (registered trademark) Boroneige SF-3 manufactured by Merck Performance Materials Co., Ltd.) was used as Comparative Example 5.

[Table 4]

| Evaluation sample | L* | | a* | | b* | | Hiding power |
|---|---|---|---|---|---|---|---|
| | White background | Black background | White background | Black background | White background | Black background | Difference in L∗ value |
| Comparative Example 1 | 84. 26 | 50.12 | 2. 83 | 0.68 | 21. 90 | 4.26 | 34. 14 |
| Comparative Example 2 | 80. 72 | 51. 68 | 3. 66 | 1. 55 | 25. 33 | 9. 79 | 29.03 |
| Example 3 | 73. 75 | 52.33 | 5. 18 | -1.56 | 35. 63 | 18. 33 | 21. 42 |
| Comparative Example 3 | 49. 58 | 45.19 | 35.76 | 24.47 | 28. 03 | 23.74 | 4.39 |
| Comparative Example 4 | 50.11 | 44. 6 | 34.6 | 21. 92 | 26. 77 | 20.88 | 5.51 |
| Example 6 | 48. 06 | 44.09 | 34.26 | 23.21 | 25.42 | 21. 33 | 3.97 |
| Comparative Example 5 | 84. 00 | 59.49 | 0. 54 | -0.23 | -1.52 | -2.15 | 24. 51 |
| Example 8 | 82. 29 | 65.18 | -0.56 | -1. 16 | -1.94 | -1.37 | 17.11 |

[Evaluation Example 7: Evaluation of tint and hiding power of particles in talc]

950 mg of talc (JA-68R manufactured by ASADA MILLING CO., LTD.), and 50 mg of any of the powders used as Comparative Examples 1 and 2 in Evaluation Example 4 or 50 mg of the particles obtained in Example 3 were added into a 10 mL volume screw cap bottle (manufactured by NICHIDEN RIKA GLASS CO,. LTD.) and were stirred by shaking. Thus, talc powder samples containing 5% of the particles were prepared.

Transparent optical adhesive sheet LUCIACS (registered trademark) CS9862UA (manufactured by NITTO DENKO CORPORATION) was attached onto microslide glass S1111 (manufactured by Matsunami Glass Ind., Ltd.). Next, the sample powder was uniformly applied with a brush onto the transparent optical adhesive sheet LUCIACS (registered trademark) CS9862UA (manufactured by NITTO DENKO CORPORATION), and the excess particles were removed with a brush so that $0.31 \text{ mg/cm}^2$ of the particles would be attached. The tint and the hiding power were measured by placing the slide glass coated with the sample onto white copy paper (A4 PPC PAPER High White) or black drawing paper PI-N86D (manufactured by MARUAI Inc.) and analyzing the sample with portable colorimeter WR10 (manufactured by Shenzhen Wave Optoelectronics Technology Co., Ltd.). The results of the measurement of L∗, a* and b* of the samples are described in Table 5. Table 5 also describes the hiding power as the difference between the L* value on the white copy paper and the L* value on the black drawing paper. The values of L∗, a* and b* and the hiding power of talc alone as a control compound were also measured.

[Table 5]

| Evaluation sample | L* | | a * | | b* | | Hiding power |
|---|---|---|---|---|---|---|---|
| | White background | Black background | White background | Black background | White background | Black background | Difference in L* value |
| Talc | 85.19 | 57.40 | 0.33 | -0.56 | -2.85 | -1.37 | 27.80 |
| Talc powder containing 5% of particles of Comparative Example 1 | 84.29 | 58.00 | 0. 29 | -0.84 | 1. 67 | 0.81 | 26.29 |
| Talc powder containing 5% of particles of Comparative Example 2 | 82.92 | 58. 24 | 0. 18 | -0.81 | 4.57 | 3.01 | 24.69 |
| Talc powder containing 5% of particles of Example 3 | 82.29 | 59.62 | 0.82 | -0.84 | 7. 84 | 4.87 | 22.68 |

[Evaluation Example 8: Evaluation of adhesion of particles]

10 mg of evaluation particles were uniformly applied with a finger (amount of application: 0.1 mg/cm$^2$) onto artificial leather Supplare (registered trademark) (manufactured by Idemitsu Technofine Co., Ltd.) that had been cut into a size of 10 cm x 10 cm. Next, PPC (copy) label, A4 size, uncut type (A-One), that had been cut into a size of 10 cm x 10 cm was placed onto the artificial leather Supplare (registered trademark) (manufactured by Idemitsu Technofine Co., Ltd.) coated with the sample powder, and was attached thereto by uniformly applying a load of 200 g. The PPC (copy) label, A4 size, uncut type (A-One) was then peeled from the artificial leather Supplare (registered trademark) (manufactured by Idemitsu Technofine Co., Ltd.), and the weight of particles adhering to the label (the amount of separated particles) was measured. The evaluation particles that were used were the particles obtained in Examples 3, 6 and 8. Further, the powders used as Comparative Examples 2 and 4 in Evaluation Example 4, and the powder used as Comparative Example 5 in Evaluation Example 6 were used as Comparative Examples. The evaluation results of the samples are described in Table 6.

[Table 6]

| Evaluation sample | Amount of separated particles (mg/cm $^2$) | Evaluation sample | Amount of separated particles (mg/cm $^2$) | Evaluation sample | Amount of separated particles (mg/cm $^2$) |
|---|---|---|---|---|---|
| Comparative Example 2 | 0. 031 | Comparative Example 4 | 0. 028 | Comparative Example 5 | 0. 027 |
| Example 3 | 0.016 | Example 6 | 0. 013 | Example 8 | 0. 020 |

[Evaluation Example 9: Evaluation of gloss]

Transparent optical adhesive sheet LUCIACS (registered trademark) CS9862UA (6 cm x 6 cm, manufactured by NITTO DENKO CORPORATION) was attached onto a glass substrate (substrate thickness: 1 mm, manufactured by TOSHIN RIKO CO., LTD.). Next, sample powder was uniformly applied with a brush onto the transparent optical adhesive sheet LUCIACS (registered trademark) CS9862UA (manufactured by NITTO DENKO CORPORATION). The evaluation particles that were used were the particles obtained in Example 8. Further, the powder used as Comparative Example 5 in Evaluation Example 7 was used as Comparative Example. The excess particles were removed with a brush so that the amount of the particles attached would be 0.2083 mg/cm$^2$ for Example 8 and 0.0694 mg/cm$^2$ for Comparative Example 5. The glass substrate coated with the sample was placed onto white copy paper (A4 PPC PAPER High White) or black drawing paper PI-N86D (manufactured by MARUAI Inc.), and the gloss was measured three times using HORIBA GLOSS CHECKER IG-320. The average values are described in Table 7 below.

[Table 7]

| Evaluation sample | White background | Black background |
|---|---|---|
| Comparative Example 5 | 30. 9 | 32. 2 |
| Example 8 | 1.7 | 1. 7 |

[Evaluation Example 10: Ignition loss test]

The particles obtained in Examples 9 and 12 to 15 were analyzed by thermogravimetric-differential thermal analysis (TG-DTA). With use of thermal analyzer Rigaku Thermoplus EVO TG8120 (manufactured by Rigaku Corporation), an aluminum pan as a sample container, and about 5 mg of aluminum oxide as the standard substance, 5 mg of the sample was heated in an air atmosphere at a heat-up rate of 10.0°C/min, and changes in weight and changes in the amount of heat were measured. The measurement results are sequentially illustrated in Figs. 13(a) to 13(e). In the figures, the weight loss at less than 100°C is ascribed to water contained in the powder, and the weight loss at about 300°C stems from the thermal decomposition of cellulose. Further, titanium oxide (MKR-1, SAKAI CHEMICAL INDUSTRY CO., LTD.) was used as Comparative Example 6; yellow iron oxide (LL-100HP, Titan Kogyo, Ltd.) as Comparative Example 7; red iron oxide (C33-8001, DIC CORPORATION) as Comparative Example 8; red iron oxide (R-516HP, Titan Kogyo, Ltd.) as Comparative Example 9; and black iron oxide (BL-100HP, Titan Kogyo, Ltd.) as Comparative Example 10. The TG-DTA measurement results of these powders are illustrated in Figs. 13(f) to 13(j), respectively.

[Evaluation Example 11: Evaluation of water content]

The particles obtained in Examples 9 to 14 were each weighed in an amount of 10 g, and the water content was measured with thermal drying moisture analyzer ML-50 (manufactured by A&D Company, Limited) under conditions in which the heating temperature was 130°C and ACCURACY: HI. The results are described in Table 8 below.

[Table 8]

| Evaluation sample | Water content |
|---|---|
| Example 9 | 1. 7% |
| Example 10 | 2. 3% |
| Example 11 | 1. 8% |
| Example 12 | 2.1% |
| Example 13 | 2. 2% |
| Example 14 | 1. 9% |

[Evaluation Example 12: Measurement of particle hardness]

The hardness of the particles obtained in Examples 9 and 12 to 15 was measured using micro compression tester MCT-510 (manufactured by Shimadzu Corporation). The measurement was performed with a test force of 4.9 mN, a load speed of 0.446 mN/sec and an upper pressure indenter of 50 $\mu$m or 20 $\mu$m. A very small amount of the sample was distributed on the lower pressure plate, and the compression test was performed with respect to individual particles. The particle hardness was calculated from the equation below as the strength C (x) at 10% deformation of the particle size. The test was carried out five times, and the results were averaged. The particle size was measured with the length measurement kit attached to the device.

$$C(x) = \frac{2.48P}{\pi d^2}$$

In the equation, P is the test force (N) at 10% deformation of the particle size, $\pi$ the pi, d the particle size (mm), and C (x) the 10% strength (MPa).
The average particle size, the test force at 10% deformation and the 10% strength of each sample are described in Table 9 below.

[Table 9]

| Evaluation sample | Average particle size ($\mu$m) | Break test force (mN) | Break strength Cs (MPa) |
|---|---|---|---|
| Example 9 | 9.83 | 0. 71 | 5. 79 |
| Example 12 | 8.82 | 0. 18 | 1. 80 |
| Example 13 | 9.44 | 0.39 | 3. 35 |
| Example 14 | 9.28 | 0.23 | 2. 04 |
| Example 15 | 9. 16 | 0.24 | 2. 22 |

[Evaluation Example 13: Measurement of pore size distribution of particles]

The pore size distribution of the particles obtained in Examples 9, 12, 13, 14 and 15 was measured using pore size distribution analyzer AutoPore V 9620 (manufactured by Shimadzu Corporation-Micromeritics). Approximately 0.17 g of the sample was added to a standard 5 cc powder cell (stem volume: 0.4 cc) and was measured under conditions in which the initial pressure was about 7 kPa (about 1 psia, corresponding to a pore diameter of about 180 $\mu$m). In the measurement, the mercury parameters were the device defaults, namely, were set at a mercury contact angle of 130° and a mercury surface tension of 485 dyne/cm.
The pore volume, the pore surface area, the median diameter, the mode diameter and the porosity of each sample are described in Table 10 below.

[Table 10]

| Evaluation sample | Pore volume (mL/g) | Pore surface area (m$^2$/g) | Median diameter ($\mu$m) | Mode diameter ($\mu$m) | Porosity (%) |
|---|---|---|---|---|---|
| Example 9 | 0. 198 | 12.6 | 0.071 | 0. 072 | 38 |
| Example 12 | 0.476 | 15.9 | 0.14 | 0. 15 | 59 |
| Example 13 | 0.251 | 11.9 | 0.11 | 0.11 | 45 |
| Example 14 | 0.485 | 13.0 | 0. 18 | 0. 19 | 61 |
| Example 15 | 0.252 | 6.2 | 0. 22 | 0. 25 | 45 |

[Evaluation Example 14: Measurement of reflected light distribution]

The particles obtained in Examples 9, 12 to 14 and 16, the powders used as Comparative Examples 6 to 9 in Evaluation Example 10, and barium sulfate (platy barium sulfate H, manufactured by SAKAI CHEMICAL INDUSTRY CO., LTD.) as Comparative Example 11 were used as samples and were analyzed in the same manner as in Evaluation Example 4 to measure the reflected light distribution and to calculate the light scattering rate. The light scattering rates of the samples at an incident light of -45 degrees are described in Table 11 below.

[Table 11]

| Results at incident light of -45° | | | |
|---|---|---|---|
| Evaluation sample | Reflection intensity at 5° angle | Reflection intensity at 20° angle | Reflection intensity at 70° angle | Light scattering rate (%) |
| Example 9 | 97. 13 | 90.44 | 36.48 | 104.05 |
| Example 12 | 86. 21 | 80.51 | 37.52 | 112.88 |
| Example 13 | 82. 03 | 74.99 | 38. 19 | 116.26 |
| Example 14 | 77.42 | 72.12 | 39. 10 | 122.93 |
| Example 16 | 52. 69 | 53. 78 | 32.90 | 145.04 |
| Comparative Example 6 | 91. 07 | 85.95 | 36.84 | 108.94 |
| Comparative Example 7 | 62. 94 | 64. 14 | 38.84 | 143.89 |
| Comparative Example 8 | 57.46 | 57.49 | 39. 75 | 153. 78 |
| Comparative Example 9 | 64. 02 | 63.02 | 39.68 | 142.44 |
| Comparative Example 11 | 30. 51 | 38.57 | 37.61 | 246.53 |

[Evaluation Example 15: Evaluation of amount of oil absorption]

The amount of oil absorption was evaluated with reference to JIS standard (K 5101-13-1: 2004 (ISO 787-5: 1980)). Linseed oil (manufactured by Summit Oil Mill) was gradually dropped with a dropper to evaluation particles that had been weighed on a weighing dish, and the mixture was kneaded together with a spatula every time the oil was dropped. The dropping was repeated until the linseed oil and the evaluation particles formed a lump. The end point of the operation was when the powder became a smooth paste that was spreadable without being cracked or collapsed and exhibited slight adhesion to a measurement plate. The amount of oil absorption (g/100 g) was calculated from the amount (g) of the linseed oil dropped relative to the weight (g) of the evaluation particles at the end point. The evaluation particles that were used were the particles obtained in Examples 9, 10, 11, 13, 14 and 15. Further, the powders used as Comparative Examples 6 to 10 in Evaluation Example 10 were provided as Comparative Examples. The results are described in Table 12 below.

[Table 12]

| Evaluation sample | Amount of oil absorption (g/100g) |
|---|---|
| Example 9 | 56.3 |
| Example 10 | 108.8 |
| Example 11 | 105.4 |
| Example 13 | 61.0 |
| Example 14 | 106.3 |
| Example 15 | 78.6 |
| Comparative Example 6 | 25.8 |
| Comparative Example 7 | 52.3 |
| Comparative Example 8 | 32.0 |
| Comparative Example 9 | 19.8 |
| Comparative Example 10 | 27.7 |

[Evaluation Example 16: Evaluation of mean coefficient of friction and mean deviation of coefficient of friction]

15 mg of sample powder was weighed and was uniformly applied onto a 5 cm x 10 cm piece of artificial leather Supplare (registered trademark) (Idemitsu Technofine Co., Ltd.). The coefficient of friction and the deviation of coefficient of friction were evaluated using friction tester KES-SE (manufactured by KATO TECH CO., LTD.) equipped with a 10 mm square silicon material sensor. The samples that were used were the particles obtained in Examples 9 to 15. Further, the powders used as Comparative Examples 6 to 10 in Evaluation Example 10 were provided as Comparative Examples. The sensor used was a 10 mm square silicon wire. The measurement distance was set to 20 mm. The static load was set to 25 gf. The measurement speed was set to 1.0 mm/sec. The contact surface width was set to 10 mm. The results are described in Table 13 below. MIU indicates the mean coefficient of friction and is an index of the slipperiness felt when a human finger touches the surface of an object. The smaller the MIU value, the higher the slipperiness. The larger the value, the lower the slipperiness. MMD indicates the mean deviation of coefficient of friction and is an index of smoothness and roughness felt when a human finger touches the surface of an object. The smaller the MMD value, the higher the smoothness. The larger the value, the greater the roughness that is felt.

[Table 13]

| Sample | MIU | MMD |
|---|---|---|
| Example 9 | 0.481 | 0.0046 |
| Example 10 | 0.343 | 0.0037 |
| Example 11 | 0.318 | 0.0050 |
| Example 12 | 0.324 | 0.0042 |
| Example 13 | 0.347 | 0.0044 |
| Example 14 | 0.333 | 0.0050 |
| Example 15 | 0.332 | 0.0042 |
| Comparative Example 6 | 0.873 | 0.0084 |
| Comparative Example 7 | 1.028 | 0.0129 |
| Comparative Example 8 | 0.749 | 0.0035 |
| Comparative Example 9 | 0.889 | 0.0068 |
| Comparative Example 10 | 0.700 | 0.0058 |

[Evaluation Example 17: Evaluation of tint and hiding power of particles]

Transparent optical adhesive sheet LUCIACS (registered trademark) CS9862UA (manufactured by NITTO DENKO CORPORATION) that had been cut into a size of 2 cm x 4 cm was attached onto microslide glass S1111 (manufactured by Matsunami Glass Ind., Ltd.). Next, evaluation particles were uniformly applied with a brush onto the transparent optical adhesive sheet LUCIACS (registered trademark) CS9862UA (manufactured by NITTO DENKO CORPORATION), and the excess particles were removed with a brush. The tint and the hiding power were measured by placing the slide glass coated with the sample onto a white background and a black background of vertical white-black hiding chart Type A (manufactured by TP Giken) and analyzing the sample with portable colorimeter WR10 (manufactured by Shenzhen Wave Optoelectronics Technology Co., Ltd.). The results of the measurement of brightness (L*), redness (a*) and yellowness (b*) of the samples are described in Table 14 below. Table 14 also describes the hiding power as the difference in L* value between on the white background and on the black background of the vertical white-black hiding chart Type A (manufactured by TP Giken) (the smaller the value, the higher the hiding power). The evaluation particles that were used were the particles obtained in Examples 9 and 12 to 16. Further, the powders used as Comparative Examples 6 to 10 in Evaluation Example 10, and the powder used as Comparative Example 11 in Evaluation Example 14 were provided as Comparative Examples.

[Table 14]

| Evaluation Sample | Weight (mg) of attached particles | White background | | | Black background | | | Hiding power |
|---|---|---|---|---|---|---|---|---|
| | | L* | a* | b* | L* | a* | b* | Δ L* |
| Blank | - | 95. 35 | 0. 28 | 2.88 | 49. 36 | 1. 89 | -1. 39 | 45. 99 |
| Example 9 | 5.0 | 87. 24 | -1. 30 | 0.37 | 81.66 | -2.29 | -1. 55 | 5.58 |
| Example 12 | 1. 7 | 67. 99 | 10.53 | 47.24 | 58. 20 | 6. 10 | 40. 59 | 9. 79 |
| Example 13 | 2.2 | 37. 96 | 39.30 | 32. 82 | 34. 27 | 37.43 | 36. 73 | 5.69 |
| Example 14 | 2.2 | 39. 20 | 39. 13 | 32.43 | 35. 31 | 37.27 | 35. 59 | 5.35 |
| Example 15 | 4.8 | 18.74 | -4.92 | 20. 80 | 18. 68 | 1. 65 | 4.91 | 0.06 |
| Example 16 | 6. 0 | 80.48 | -0.52 | 1. 75 | 63. 15 | -0.52 | -0. 19 | 17.33 |
| Comparative Example 6 | 0.8 | 80.45 | -0.52 | -0.11 | 65. 15 | -3. 70 | -6. 26 | 15.30 |
| Comparative Example 7 | 0.2 | 80. 87 | 3.43 | 31. 48 | 49. 33 | -0.15 | 11. 82 | 31. 54 |
| Comparative Example 8 | 0.7 | 52. 54 | 38.46 | 30. 50 | 39.48 | 17.04 | 15. 03 | 13. 06 |
| Comparative Example 9 | 0.5 | 51. 38 | 37.33 | 27. 06 | 42.81 | 21. 03 | 17.95 | 8.57 |
| Comparative Example 10 | 1.0 | 26.74 | -1. 59 | 8.29 | 26. 15 | -2.99 | 8.99 | 0.59 |
| Comparative Example 11 | 3.6 | 86. 13 | 0. 15 | 2.45 | 58. 64 | 0.59 | 0. 15 | 27.49 |

[Evaluation Example 18: Evaluation of angle dependence of tint]

The angle dependence of the tint of O/W foundation samples containing evaluation particles was evaluated using gonio-spectrophotometer GCMS-4 (manufactured by MURAKAMI COLOR RESEARCH LABORATORY CO., LTD.). D65 was used as a light source, and the measurement was performed at an incident angle of 0°, a light receiving angle of 10° to 80°, and a pitch angle of 5°.

The measurement results were quantified by the L*a*b* color system (JIS Z 8781-4) for color quantification. L* indicates brightness, a∗ redness, and b∗ yellowness. The difference in tint between two points having different L∗a∗b∗ color spaces is determined as the color difference ΔEab by calculating the differences in L*, a*, b* between the two different points, combining the square values of these differences, and taking the square root of the sum. Here, the angle dependence of tint was evaluated by determining the color difference ΔEab of the tint at a light receiving angle from the tint at a light receiving angle of 10° as the standard, and calculating the average of the color differences (reference was made to Japanese Patent Application Kokai Publication No. 2018-97320).

The O/W foundation sample containing evaluation particles was applied with a four-sided applicator onto a black background of vertical white-black hiding chart Type A (manufactured by TP Giken) to form a coating film (thickness: 100 μm). The film was dried at room temperature and was bonded to an acrylic plate with a double-sided tape. An evaluation sample was thus fabricated.

The composition of the O/W foundation sample formulation used in the evaluation is described in Table 15 below. The ingredients in columns A and B were separately heated to about 80°C and were combined together by being stirred with a homomixer at 7000 rpm for 3 minutes. Thereafter, the mixture was cooled to room temperature while performing stirring with a propeller stirrer at 300 rpm. When the temperature returned to room temperature, the ingredients in column C were added, and stirring was continued for another 10 minutes. To 54.0 g of this formulation, 6.0 g of evaluation particles were added. The resultant mixture was stirred and mixed together with a homomixer at 5000 rpm for 2 minutes to give an O/W foundation sample. The evaluation particles that were used were the particles obtained in Examples 9, 12, 13 and 14. Further, O/W foundation samples were obtained by adding 4.8 g of comparative particles to 55.2 g of the above formulation, and stirring and mixing the mixture with a homomixer at 5000 rpm for 2 minutes. The comparative particles that were used were the powders used as Comparative Examples 6 to 9 in Evaluation Example 10. The results are described in Table 16 below.

[Table 15]

| | Ingredients | Manufacturers | Composition (%) |
|---|---|---|---|
| A | Behenyl alcohol (NIKKOL NIKKOMULESE 41) | Nikko Chemicals Co. , Ltd. | 5.0 |
| | Macadamia seed oil Jojoba seed oil Olive fruit oil Rosa canina fruit oil Squalane (NIKKOL NATURAL OILS-1) | Nikko Chemicals Co., Ltd. | 7.0 |
| | Ethylhexylglycerin Glyceryl caprylate (NIKKOL NIKKOGUARD 88) | Nikko Chemicals Co., Ltd. | 0.5 |
| | Dipropylene glycol (DPG-RF) | ADEKA CORPORATION | 5.0 |
| | Concentrated glycerin | Sakamoto Yakuhin Kogyo Co., Ltd. | 5.0 |
| B | Purified water | - | 75.0 |
| | Stearoylmethyltaurine Na (NIKKOL SMT) | Nikko Chemicals Co., Ltd. | 0. 1 |
| | EDTA-2Na | JUNSEI CHEMICAL CO., LTD. | 0. 1 |
| | Hydroxyethyl cellulose | NACALAI TESQUE, INC. | 0. 1 |
| | Xanthan gum | SANSHO Co., Ltd. | 0.2 |
| C | Polyglyceryl-10 laurate Glycerin Dimethicone Water (NIKKOL NET-813-1) | Nikko Chemicals Co., Ltd. | 2.0 |

[Table 16]

| Evaluation sample | Average of color differences ΔEab of tints at light receiving angles of 15° to 80° from tint at light receiving angle of 10° as standard color |
|---|---|
| Example 9 | 3. 26 |

(continued)

| Evaluation sample | Average of color differences ∆Eab of tints at light receiving angles of 15° to 80° from tint at light receiving angle of 10° as standard color |
|---|---|
| Example 12 | 8. 92 |
| Example 13 | 20. 59 |
| Example 14 | 6. 19 |
| Comparative Example 6 | 11.37 |
| Comparative Example 7 | 19.70 |
| Comparative Example 8 | 38.71 |
| Comparative Example 9 | 26. 22 |

[Evaluation Example 19: Determination of distribution of cellulose and iron oxide in particles]

Silver paste was applied onto a silicon wafer, and the particles obtained in Example 12 were sprinkled. Next, the excess particles were removed by air blowing, and platinum was deposited with ion sputter MC1000 (manufactured by Hitachi, Ltd.) at 15 mA for 100 seconds. A sample was thus fabricated. A slice of this sample was prepared using focused ion beam-scanning electron microscope (FIB-SEM) Helios NanoLab G3 (manufactured by Thermo Fisher Scientific) and was observed with multifunction electron microscope JEM-F200 (manufactured by JEOL Ltd.) at an acceleration voltage of 200 kV. The transmission electron microscope (TEM) images obtained are illustrated in Figs. 14(a) to 14(c). Further, the distribution of carbon (C), oxygen (O) and iron (Fe) in the cross section, and an EDX spectrum in the measurement region were evaluated by energy dispersive X-ray analysis (TEM-EDX). The image of the analyzed region, and the images of distribution of carbon (C), oxygen (O) and iron (Fe) are illustrated in Figs. 15(a) to 15(d), respectively. The EDX spectrum obtained is illustrated in Fig. 16.

[Formulation Example 1: O/W foundation]

The formulation composition is described in Table 17 below. The ingredients in columns A and B were separately heated to about 80°C and were combined together by being stirred with a homomixer at 7000 rpm for 3 minutes. Thereafter, the mixture was cooled to room temperature while performing stirring with a propeller stirrer at 300 rpm. When the temperature returned to room temperature, the ingredient in column C was added, and stirring was continued for another 10 minutes. To this formulation, the particles obtained in Examples 9, 12, 14 and 15 were added. The resultant mixture was stirred and mixed together with a homomixer at 5000 rpm for 2 minutes to give an O/W foundation.

[Table 17]

| | Ingredients | Manufacturers | Composition (%) |
|---|---|---|---|
| A | NIKKOL NIKKOMULESE 41 | Nikko Chemicals Co., Ltd. | 4. 35 |
| | NIKKOL NATURAL OILS-1 | Nikko Chemicals Co., Ltd. | 6.09 |
| | NIKKOL NIKKOGUARD 88 | Nikko Chemicals Co., Ltd. | 0.43 |
| | DPG-RF | ADEKA CORPORATION | 4.35 |
| | Concentrated glycerin | Sakamoto Yakuhin Kogyo Co., Ltd. | 4.35 |
| B | Purified water | - | 65. 22 |
| | NIKKOL SMT | Nikko Chemicals Co., Ltd. | 0.09 |
| | EDTA-2Na | JUNSEI CHEMICAL CO., LTD. | 0.09 |
| | Hydroxyethyl cellulose | NACALAI TESQUE, INC. | 0.09 |
| | Xanthan gum | SANSHO Co., Ltd. | 0. 17 |
| C | NIKKOL NET-813-1 | Nikko Chemicals Co., Ltd. | 1. 74 |

(continued)

|  | Ingredients | Manufacturers | Composition (%) |
|---|---|---|---|
| D | Example 9 | - | 11.21 |
|  | Example 12 |  | 1. 38 |
|  | Example 14 |  | 0.37 |
|  | Example 15 | - | 0. 10 |

[Formulation Example 2: Pressed foundation]

The formulation composition is described in Table 18 below. The ingredients in column A were stirred and mixed together with a sample mill (SK-M10 manufactured by Kyoritsu Riko) for 30 seconds. The ingredients in column B were added. The mixture was lightly mixed with a spatula and was thereafter stirred and mixed with the sample mill for 5 seconds three times. 13 g of the powder was weighed on an aluminum dish and was compacted with a press machine at 7 MPa to give a pressed foundation.

[Table 18]

|  | Ingredients | Manufacturers | Composition (%) |
|---|---|---|---|
| A | Talc JA-68R | ASADA MILLING CO., LTD. | 31. 48 |
|  | Sericite FSE | Sanshin Mining Ind. Co., Ltd. | 19. 36 |
|  | Mica Y2300X | YAMAGUCHI MICA C0., LTD. | 9.68 |
|  | Example 9 | - | 9.68 |
|  | Example 12 |  | 6.02 |
|  | Example 14 | - | 1. 74 |
|  | Example 15 | - | 0.37 |
|  | (Vinyl dimethicone/methicone silsesquioxane) crosspolymer (KSP-100) | Shin-Etsu Chemical Co., Ltd. | 0.97 |
|  | Polymethyl methacrylate microparticles (Matsumoto Microsphere M-100) | Matsumoto Yushi-Seiyaku Co., Ltd. | 5.81 |
|  | Titanium oxide microparticles (MT-100TV) | TAYCA Co., Ltd. | 4.84 |
|  | Ethylparaben | UENO FINE CHEMICALS INDUSTRY, LTD. | 0. 39 |
| B | Methyl phenyl silicone oil (KF-56A) | Shin-Etsu Chemical Co., Ltd. | 0.97 |
|  | Dimethicone (KF-96-6) | Shin-Etsu Chemical Co., Ltd. | 0.97 |
|  | Diisostearyl malate (COSMOL 222) | The Nisshin OilliO Group, Ltd. | 1. 94 |
|  | Triethylhexanoin (TIO) | The Nisshin OilliO Group, Ltd. | 1. 94 |
|  | Sorbitan sesquiisostearate (COSMOL 182V) | The Nisshin OilliO Group, Ltd. | 0.97 |
|  | Octyl methoxycinnamate | The Nisshin OilliO Group, Ltd. | 2.90 |

[Formulation Example 3: Oil-based foundation]

The formulation composition is described in Table 19 below. The ingredients in column A were stirred and mixed together with a sample mill (SK-M10 manufactured by Kyoritsu Riko) for 5 seconds three times. The ingredients in column B were uniformly dissolved by being heated to about 85°C, and the mixture of the ingredients in column A was added thereto. The resultant mixture was mixed with a spatula. The mixture liquid was stirred and mixed with a homodisper at 2,500 rpm for 1 minute, added into a container, and cooled to room temperature. An oil-based foundation was thus obtained.

[Table 19]

|  | Ingredients | Manufacturers | Composition (%) |
|---|---|---|---|
| A | Sericite FSE | Sanshin Mining Ind. Co., Ltd. | 24.69 |
| | Example 9 | - | 14.85 |
| | Example 12 | - | 0.63 |
| | Example 14 | - | 2.24 |
| | Example 15 | - | 0. 15 |
| B | COSMOL 222 | The Nisshin OilliO Group, Ltd. | 9.50 |
| | α-Olefin oligomer | The Nisshin OilliO Group, Ltd. | 14.25 |
| | Cyclopentasiloxane (KF-995) | Shin-Etsu Chemical Co., Ltd. | 16. 15 |
| | Polyethylene wax (PERFORMALENE PL) | New Phase Technologies | 1. 52 |
| | Carnauba wax | TOA KASEI CO., LTD. | 0.95 |
| | CERESIN C | NIKKO RICA CORPORATION. | 3.33 |
| | Microcrystalline wax | NIPPON SEIRO C0., LTD. | 0.86 |
| | COSMOL 182V | The Nisshin OilliO Group, Ltd. | 1. 14 |

Industrial Applicability

The porous pigment particles of the present invention are resistant to aggregation and are excellently dispersed in a base material to impart a color while improving the dullness problem. The particles of the present invention are porous particles that contain cellulose and have a wrinkle-like or fold-like uneven structure on the surface thereof (that is, have an appropriate amount of pores or voids). Thus, the particles of the present invention have soft and comfortable texture and are suitably added to cosmetics that are directly applied to the skin. Further, the particles of the present invention have excellent optical characteristics (light scattering properties) in which incident light is uniformly scattered, and are therefore expected to produce a defocus effect (or a soft focus effect), finding suitable use in the field of cosmetics that are directly applied to the skin.

**Claims**

1. A porous pigment particle comprising cellulose or a cellulose derivative, and an inorganic pigment as main components.

2. The particle according to Claim 1, which has a void ratio in the range of 5 to 50%.

3. The particle according to Claim 1 or 2, which has a particle size in the range of 0.1 to 500 μm.

4. The particle according to any one of Claims 1 to 3, which has a light scattering rate in the range of 50 to 230%.

5. The particle according to any one of Claims 1 to 4, wherein, in a measurement of a distribution of reflected light, a ratio of an intensity of the reflected light in a vicinity of a specular reflection to incident light with respect to an intensity of the reflected light in a vicinity of the incident light is in the range of 0.5:1 to 2:1.

6. The particle according to any one of Claims 1 to 5, wherein the cellulose is crystalline cellulose.

7. The particle according to any one of Claims 1 to 6, wherein the inorganic pigment is at least one selected from the group consisting of white pigments, color pigments, pearlescent pigments and functional pigments.

8. The particle according to any one of Claims 1 to 7, wherein the inorganic pigment is selected from the group consisting of red iron oxide, yellow iron oxide, black iron oxide, chromium oxide, ultramarine blue, Prussian blue, manganese purple, titanium oxide, zinc oxide, aluminum oxide, zirconium oxide, boron nitride and barium sulfate.

9. The particle according to any one of Claims 1 to 8, wherein the ratio (by weight) of the cellulose or the cellulose derivative to the inorganic pigment is in the range of 1:1 to 1:20.

10. A method for producing a porous pigment particle containing cellulose or a cellulose derivative, and an inorganic pigment as main components, the method comprising a step of obtaining a dispersion containing cellulose or a cellulose derivative, and an inorganic pigment, and a step of spray-drying the dispersion obtained.

11. The production method according to Claim 10, wherein the dispersion is obtained through physical pulverization of the cellulose or the cellulose derivative, and the inorganic pigment.

12. The production method according to Claim 10 or 11, wherein the concentration of solids containing the cellulose or the cellulose derivative, and the inorganic pigment in the dispersion is 0.5 to 50 mass%.

13. The production method according to any one of Claims 10 to 12, wherein the ratio (by weight) of the cellulose or the cellulose derivative to the inorganic pigment is in the range of 1:1 to 1:20.

14. A cosmetic comprising the particle described in any one of Claims 1 to 9, or a particle obtained by the production method described in any one of Claims 10 to 13.

Fig. 1 (a)

Fig. 1 (b)

Fig. 1 (c)

Fig. 1 (d)

Fig. 1 (e)

Fig. 1 (f)

Fig. 1 (g)

Fig. 1 (h)

Fig. 1 (i)

Fig. 1 (j)

Fig. 1 (k)

Fig. 1 (l)

Fig. 1 (m)

Fig. 1 (n)

Fig. 1 (o)

Fig. 1 (p)

Fig. 1 (q)

Fig. 1 (r)

Fig. 1 (s)

Fig. 1 (t)

Fig. 1 (u)

Fig. 2 (a)

Fig. 2 (b)

Fig. 2 (c)

Fig. 2 (d)

Fig. 2 (e)

Fig. 2 (f)

Fig. 2 (g)

Fig. 2 (h)

Fig. 2 (i)

Fig. 2 (j)

Fig. 2 (k)

Fig. 2 (l)

Fig. 2 (m)

Fig. 2 (n)

Fig. 2 (o)

Fig. 2 (p)

Fig. 3

(a)

(b)

ENTIRE SECTIONAL AREA OF PARTICLE : $176.4$ $(\mu m^2)$

(c)

TOTAL VOID AREA : $18.17$ $(\mu m^2)$

# Fig. 4

(a)

(b)

ENTIRE SECTIONAL AREA OF PARTICLE : 3 4 . 2 7 $(\mu m^2)$

(c)

TOTAL VOID AREA : 7 . 4 3 $(\mu m^2)$

Fig. 5

（a）

（b）

ENTIRE SECTIONAL AREA OF PARTICLE： 4 2． 2 4 （$\mu$ m$^2$）

（c）

TOTAL VOID AREA： 8． 9 9 （$\mu$ m$^2$）

Fig. 6

(a)

(b)

ENTIRE SECTIONAL AREA OF PARTICLE: 7 2. 7 3 ($\mu$m$^2$)

(c)

TOTAL VOID AREA: 1 4. 3 7 ($\mu$m$^2$)

## Fig. 7

(a)

(b)

ENTIRE SECTIONAL AREA OF PARTICLE : $33.97$ $(\mu m^2)$

(c)

TOTAL VOID AREA : $8.28$ $(\mu m^2)$

Fig. 8

(a)

(b)

ENTIRE SECTIONAL AREA OF PARTICLE : 4 6. 9 3 ($\mu$ m$^2$)

(c)

TOTAL VOID AREA : 7. 0 7 ($\mu$ m$^2$)

Fig. 9

(a)

(b)

ENTIRE SECTIONAL AREA OF PARTICLE : $2\,9\,9.\,9\,9$ $(\mu\,\mathrm{m}^{2})$

(c)

TOTAL VOID AREA : $5\,2.\,2\,5$ $(\mu\,\mathrm{m}^{2})$

Fig. 10

(a)

(b)

ENTIRE SECTIONAL AREA OF PARTICLE : 1 8 5 ． 0 5 （$\mu$ m$^2$）

(c)

TOTAL VOID AREA : 4 2 ． 2 5 （$\mu$ m$^2$）

Fig. 11

(a)

(b)

ENTIRE SECTIONAL AREA OF PARTICLE : 6 9 . 9 8 ($\mu$ m$^2$)

(c)

TOTAL VOID AREA : 1 5 . 7 8 ($\mu$ m$^2$)

## Fig. 12

(a)

(n)

ENTIRE SECTIONAL AREA OF PARTICLE: 1 8 4 . 4 7 ($\mu$m$^2$)

(o)

TOTAL VOID AREA : 3 7 . 2 ($\mu$m$^2$)

Fig. 13 (a)

Fig. 13 (b)

Fig. 13 (c)

Fig. 13 (d)

Fig. 13 (e)

Fig. 13 (f)

Fig. 13 (g)

Fig. 13 (h)

Fig. 13 (i)

**EP 4 006 110 A1**

Fig. 13 (j)

Fig. 14 (a)

Fig. 14 (b)

Fig. 14 (c)

Fig. 15

(a) EDX ANALYZED REGION

(b) IMAGE OF CARBON (C) DISTRIBUTION

200 nm    SEI(frame1)

200 nm    C K

(c) IMAGE OF OXYGEN (O) DISTRIBUTION

(d) IIMAGE OF IRON (Fe) DISTRIBUTION

200 nm    O K

200 nm    Fe K

Fig. 16

**INTERNATIONAL SEARCH REPORT**

| International application No. |
| --- |
| PCT/JP2020/029440 |

### A. CLASSIFICATION OF SUBJECT MATTER

C09C 1/00(2006.01)i; C09C 1/02(2006.01)i; C09C 1/04(2006.01)i; C09C 1/24(2006.01)i; C09C 1/26(2006.01)i; C09C 1/32(2006.01)i; C09C 1/34(2006.01)i; C09C 1/36(2006.01)i; C09C 1/40(2006.01)i; C09C 3/10(2006.01)i; A61Q 1/00(2006.01)i; A61Q 17/04(2006.01)i; A61Q 19/00(2006.01)i; A61Q 19/10(2006.01)i; A61Q 5/00(2006.01)i; A61K 8/02(2006.01)i; A61K 8/19(2006.01)i; A61K 8/26(2006.01)i; A61K 8/27(2006.01)i; A61K 8/28(2006.01)i; A61K 8/29(2006.01)i; A61K 8/73(2006.01)i

FI: C09C1/00; C09C1/02; C09C1/26; C09C1/24; C09C1/36; C09C1/34; C09C1/40; C09C1/04; A61K8/02; A61K8/19; C09C1/32; A61K8/29; A61K8/28; A61K8/26; A61Q1/00; C09C3/10; A61Q19/00; A61Q19/10; A61Q5/00; A61Q17/04; A61K8/27

According to International Patent Classification (IPC) or to both national classification and IPC

### B. FIELDS SEARCHED

Minimum documentation searched (classification system followed by classification symbols)

C09C1/00; C09C1/02; C09C1/04; C09C1/24; C09C1/26; C09C1/32; C09C1/34; C09C1/36; C09C1/40; C09C3/10; C09C1/00; A61Q17/04; A61Q19/00; A61Q19/10; A61Q5/00; A61K8/02; A61K8/19; A61K8/26; A61K8/27; A61K8/28; A61K8/29; A61K8/73

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

CAplus/REGISTRY (STN); JSTPlus/JMEDPlus/JST7580 (JDreamIII); Science Direct; Japio-GPG/FX

### C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 52-71526 A (DAI NIPPON TORYO CO., LTD.) 15 June 1977 (1977-06-15) claims, page 2, lower left column, line 10 to page 2, lower right column, line 12, example 1 | 1-13<br>14 |
| X<br>Y | JP 63-110261 A (PENTEL CO., LTD.) 14 May 1988 (1988-05-14) claims, page 2, upper left column, line 8 to page 2, lower left column, line 6, example 2, effect of the invention | 1, 3, 7-9, 14<br>14 |

☒ Further documents are listed in the continuation of Box C.      ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 08 October 2020 (08.10.2020) | 20 October 2020 (20.10.2020) |

| Name and mailing address of the ISA/<br>Japan Patent Office<br>3-4-3, Kasumigaseki, Chiyoda-ku,<br>Tokyo 100-8915, Japan | Authorized officer |
| --- | --- |
| | Telephone No. |

Form PCT/ISA/210 (second sheet) (January 2015)

| | International application No. |
|---|---|
| | PCT/JP2020/029440 |

C (Continuation).    DOCUMENTS CONSIDERED TO BE RELEVANT

| Category* | Citation of document, with indication, where appropriate, of the relevant passages | Relevant to claim No. |
|---|---|---|
| X<br>Y | JP 2007-56086 A (DAITO KASEI KOGYO CO., LTD.) 08 March 2007 (2007-03-08) claims 1-5, 7, examples, fig. 1-2 | 1, 3, 7-9, 14<br>14 |
| A | JP 2013-216631 A (ONO, Mamoru) 24 October 2013 (2013-10-24) examples | 1-14 |
| A | JP 2004-501215 A (TIMSON OY) 15 January 2004 (2004-01-15) examples | 1-14 |
| A | NEVES, M. C. et al., "Eco-friendly hybrid pigments made of cellulose and iron oxides", Journal of Nanoscience and nanotechnology, August 2012, vol. 12, pp. 1-5, abstract | 1-14 |
| A | ZHANG, X. et al., "Building dual-scale roughness using inorganic pigments for fabrication of superhydrophobic paper", Industrial and Engineering Chemistry Research, 14 March 2017, vol. 56, pp. 3618-3628, abstract | 1-14 |
| P, A | WO 2019/151486 A1 (NISSAN CHEMICAL CORPORATION) 08 August 2019 (2019-08-08) claims | 1-14 |

Form PCT/ISA/210 (continuation of second sheet) (January 2015)

INTERNATIONAL SEARCH REPORT

Information on patent family members

International application No.

PCT/JP2020/029440

| Patent Documents referred in the Report | Publication Date | Patent Family | Publication Date |
|---|---|---|---|
| JP 52-71526 A | 15 Jun. 1977 | (Family: none) | |
| JP 63-110261 A | 14 May 1988 | (Family: none) | |
| JP 2007-56086 A | 08 Mar. 2007 | (Family: none) | |
| JP 2013-216631 A | 24 Oct. 2013 | (Family: none) | |
| JP 2004-501215 A | 15 Jan. 2004 | US 2004/0202835 A1 examples US 2005/0221076 A1 examples WO 2001/079606 A1 examples EP 1276932 A1 examples | |
| WO 2019/151486 A1 | 08 Aug. 2019 | TW 201940521 A claims | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010092890 A

- JP 2018097320 A, Kokai